(19) Europäisches Patentamt

European Patent Office

Office européen des brevets

(11) **EP 1 973 529 B1**

(12) **EUROPEAN PATENT SPECIFICATION**

(45) Date of publication and mention
of the grant of the patent:
**25.05.2011 Bulletin 2011/21**

(51) Int Cl.:
***A61K 9/20*** *(2006.01)*

(21) Application number: **07700149.3**

(86) International application number:
**PCT/DK2007/000008**

(22) Date of filing: **05.01.2007**

(87) International publication number:
**WO 2007/076874 (12.07.2007 Gazette 2007/28)**

(54) **DISINTEGRATING LOADABLE TABLETS**

ZERFALLENDE BEFÜLLBARE TABLETTEN

COMPRIMÉS DÉSINTÉGRABLES POUVANT ÊTRE CHARGÉS

(84) Designated Contracting States:
**AT BE BG CH CY CZ DE DK EE ES FI FR GB GR HU IE IS IT LI LT LU LV MC NL PL PT RO SE SI SK TR**

(30) Priority: **05.01.2006 DK 200600028**

(43) Date of publication of application:
**01.10.2008 Bulletin 2008/40**

(73) Proprietor: **LifeCycle Pharma A/S**
**2970 Horsholm (DK)**

(72) Inventors:
• **HOLM, Per**
**2720 Vanløse (DK)**

• **SLOT, Lillian**
**2830 Virum (DK)**

(74) Representative: **Joersboe, Anne-Marie Charlotte H.**
**LifeCycle Pharma A/S**
**Kogle Allé 4**
**2970 Harsholm (DK)**

(56) References cited:
**EP-A1- 0 644 156          WO-A-00/38655**
**WO-A-03/063831          WO-A-2005/051358**
**US-A1- 2002 086 055     US-A1- 2004 253 312**
**US-A1- 2005 019 398     US-B1- 6 399 591**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

### Field of the invention

The present invention relates to a novel disintegrating tablet product in compressed form that in an easy, flexible and reproducible manner can be loaded with a relatively high amount of a pharmaceutically acceptable liquid formulation e.g. carrying a therapeutically, prophylactically and/or diagnostically active substance. The novel tablet product may be produced in large- scale batches and stored until use and each batch or sub-batch may be loaded with the same or different pharmaceutically acceptable liquid formulations and/or active substances. The invention also provides tablets that have been loaded with such a liquid formulation as well as a method for the preparation thereof.

The invention provides a means for obtaining tablets comprising an active substance suitable for providing a substantial fast release by incorporation of disintegrating excipients in order to influence the accessibility of the active substance e.g. to be released and/or absorbed upon oral administration. The invention is highly suitable for the loading of tablets with substances having a low water solubility and especially in such cases where the substance is desired to be delivered in microcrystalline and/or amorphous form to increase release and absorption.

Accordingly, the invention provides a means for obtaining tablets comprising an active substance together with a suitable and relatively high amount of a liquid that influences the accessibility of the active substance e.g. to be released and/or absorbed upon oral administration.

### Background of the invention

Many drug substances have and it is expected that many of the future drug substances will have undesired properties especially with respect to e.g. water solubility and to oral bioavailability. Therefore, novel technologies, which enable especially therapeutically and/or prophylactically active substances to be delivered to the body in a relatively easy manner and at the same time enables the desired therapeutic and/or prophylactic response, is highly needed.

In the pharmaceutical area it is common to prepare pharmaceutical compositions comprising one or more active substances and various excipients. One reason for preparing such pharmaceutical compositions is to manipulate the availability of the active compound after ingestion of the pharmaceutical composition.

For the preparation of pharmaceutical composition for oral administering the active substances are often incorporated into an agglomerated preparation in order to provide the active compounds in a form that may be pressed into tablets or filled into capsules.

Beside providing the active substance in a form that may be pressed into tablets, agglomerates may also be designed to secure a desired availability of the active compound after ingestion of a pharmaceutical composition containing said granule.

The enhancement of oral bioavailability of poorly water soluble drugs as well as providing a fairly water soluble drug in a sustained release form remain one of the most challenging aspects of drug development and further development of the agglomeration techniques may provide valuable tools for these aspects.

One commonly used technique for granulation is wet granulation, where a mixture of powders including the active substance is mixed with a liquid, usually an aqueous medium, under mechanical influence for the preparation of granules. Usually the granules prepared by wet granulation are dried before use.

Melt agglomeration and controlled agglomeration are techniques for agglomeration of an active substance, essentially performed by melting a pharmaceutical acceptable vehicle such as an oil or an oily-like material, dissolution or dispersion of one or more active compounds in the melted vehicle and deposition of the thus prepared mixture on a particulate material, the filler, and subsequently the particles adhere to each other and form agglomerates.

In WO 03/004001 (by the present applicant) is described a novel technique of controlled agglomeration by which it is possible to load a particulate material with a relatively high amount of an oil or an oily-like material. The technique is based on a process that involves spraying of a carrier composition containing the oil or oily-like material onto a particulate material. The process conditions enable the particulate material to be loaded with a relatively high amount of the oil or oily-like material. Normally, the process involves heating of the carrier composition and maintaining the temperature of the carrier composition during application. As the application is performed by spraying, strict temperature control of the spraying equipment is a requirement in order to avoid problems relating to clotting of the spray nozzle etc.

### Description of the invention

The present inventors have now found a much more simple solution. They have found that it is possible to prepare a tablet solely containing inert pharmaceutically acceptable excipients (although in some cases it may be suitable also to incorporate an active substance therein) and when the tablet is subjected to a pharmaceutically acceptable liquid formulation e.g. containing the active substance, the tablet will due to its porosity - suck the liquid formulation into the tablet. Most surprising this loading of an inert tablet takes place within a relatively short period of time and is reproducible, i.e. the same amount of liquid formulation is sorbed when the same type and size of tablet and liquid formulation is used (see the examples herein).

Moreover, the inventors have observed that it is important to incorporate one or more disintegrants in the tablet in order to ensure a sufficient release and/or release rate of an active substance that subsequently may be loaded into the tablet.

Incorporation of one or more disintegrants is of particular importance in order to ensure a relatively fast and/or essentially complete release of the active substance. It is envisaged that incorporation of one or more disintegrants does not substantially influence the loading capability of the porous tablet, especially as the concentration of the one or more disintegrants that is necessary in order to achieve the desired effect is relatively low.

Previously, the inventors believed that incorporation of one or more superdisintegrants into specific porous tablet compositions did not seem to substantially influence the release of the active substance. However, as demonstrated in the examples herein, the picture has been found to be much more complex. Thus, e.g., the disintegration time of a porous tablet loaded with a liquid composition is very much dependent on the specific type of liquid employed, and the use of disintegrants including superdisintegrants optionally in combination with one or more hydrophilic substances that may facilitate wetting of the tablet seem to have marked influence on the release rate of an active substance that has been loaded into a porous tablets by means of a liquid composition.

Accordingly, the present invention is based on the observation that incorporation of one or more disintegrants is important in order to facilitate a relatively fast release of the active substance from the loaded tablet. Moreover, in specific embodiments, incorporation of a hydrophilic agent such as a wetting agent or a humectant enables an impact of water on the tablet and possibly contributes to a faster disintegration and/or release of the active substance from the tablet. To the best of the inventors' knowledge, inert tablets with the above-mentioned properties have not been recognized or used before in the pharmaceutical field to load tablets with liquids e.g. containing an active substance.

US 2002/0086055 (Alza Corporation) relates to formulations where a pharmaceutical liquid formulation is provided in solid dosage forms by dispersing porous particles comprising the liquid into osmotic push layer dosage forms. There is no disclosure of a loadable compressed tablet.

WO 00/38655 (Alza Corporation) relates to dosage forms comprising a plurality of particles. The particles are filled porous particles where the composition used for filling the pores of the porous particles is a liquid active agent formulation adapted to be retained in the pores during a compacting process. There is no disclosure of a loadable compressed tablet.

WO 002005/051358 (Alza Corporation) relates to a drug delivery system where a drug is complexed with a polymer and contacted with a porous carrier where the drug polymer complex will disassociate from the carrier upon contact with water. Tablets are formed by conventional loading with drug by mixing or granulation before compressing into tablets and it is further suggested that the formulation may be used with the ALZA OROS™ PUSH-PULL system in accordance with the disclosure of US 2002/0086055 and WO 00/38655 above also from Alza Corporation. There is no disclosure of a loadable compressed tablet.

US 6,399,591 relates to blank tablets comprising an absorbent material in concentrations of 0.1 to 5% and up to 98% of a diluent or binder and up to 10% of a disintegrant. In the five examples, the absorbent is used in a concentration of 1.25% or 1.53% and a load up to 14%. Accordingly, there is no teaching of a highly porous compressed tablet according to the present invention.

US 2005/0019398 relates to a flash melt oral dosage form comprising super disintegrants and dispersing agents having surface areas in the range from 0.98 to 209 m2/g. There is no disclosure of a loadable compressed tablet.

US 2004/0253312 relates to a oral dosage form with a solid core and a shell comprising one or more openings. It is mentioned that the core has a porosity of less then 40%, preferable 30%. The tablet cores are made by mixing the ingredients with the active substance before compression. There is no disclosure of a loadable compressed tablet.

WO 03/063831 relates to the production of tablets with a high load of low solubility active ingredient. The high load is obtained by preparing an amorphous solid dispersion of the drug including a concentration enhancing polymer. It is mentioned that solid amorphous dispersion has a tendency to undergo plastic deformation when compressed into tablets which can lead to unacceptable low tablet porosity. In order to ensure the tablet has sufficient porosity to allow adequate wicking of water into the tablet to cause rapid tablet disintegration, a porosity of at least 0.15 is obtained by including porosigens to the composition before compressing. There is no disclosure of a loadable compressed tablet.

The tablets provided by the present invention can be loaded with any type of active substance as well as they can be designed to any type of release of the active substance. In all cases the active substance must be in liquid form. The active substance itself may be liquid and/or it may be dispersed or dissolved in a suitable medium before loading the tablet with the active substance.

The loading of the inert tablets is dependent on the type, nature and concentration of the sorbent material that are contained in the tablet. The critical parameter is, however, not only the properties of the sorbent material contained in the tablet, but also the properties of the tablet itself. To this end, the most critical properties are the ability of the tablet i) to sorb a pharmaceutically acceptable liquid formulation in a sufficient amount, ii) to maintain the amount sorbed during storage without any sweating of the liquid formulation from the surface of the tablet, and iii) to release the active substance once the tablet is subject to an in vitro dissolution test and/or is administered orally to a subject such as an animal including a human. In the present context, the term "sorb" is intended to include absorb as well as adsorb and, accordingly, a sorbent material includes an absorbent material as well as an adsorbent material.

To fulfill these requirements, the present inventors have identified that one of the critical property of the tablets to be loaded is the porosity of the tablet.

Thus, the present invention relates to a disintegrating loadable tablet product in compressed form comprising

i) at least 60% w/w of a sorbent material having a specific surface area (BET surface area) of at least 50 m$^2$/g as measured by gas adsorption or mixtures of such sorbent materials, and
ii) a disintegrant or a mixture of disintegrants,

wherein the tablet in compressed form has

a) a porosity of 45% v/v or more,
b) a hardness of at least 20 Newton, and
c) a loading capacity of at least 30% of a liquid substance or liquid composition.

Accordingly, in one aspect, the present invention relates to a disintegrating loadable tablet having a porosity of 45 % v/v or more as a pharmaceutical carrier composition for a pharmaceutically acceptable liquid formulation. The normal tablets used within the pharmaceutical field have a porosity that is much lower. One of the reasons to avoid very porous tablets are that such tablets do not have sufficient robustness to enable the normal handling of tablets during packaging and storage, i.e. they are excepted not to fulfill the pharmacopoeia requirements with respect to hardness and friability. Moreover, another critical parameter is the ability and feasibility of a tablet loaded with an active substance to release the active substance from the tablet. To this end, a critical parameter seems to be the presence of a disintegrant in the tablet.

Porosity is defined as the volume ratio between the voids in the tablet and the total volume of the tablet according to Equation 1 in the Examples herein.

The term "loading capacity" is defined in Example 1 herein.

**Loadable tablets**

In the present context the term "inert tablet" is used to denote a tablet that solely contains ingredients that normally are regarded as inert with respect to therapeutic effect. More specifically, such a tablet contains pharmaceutically acceptable excipients selected from the group consisting of fillers, diluents, binders, lubricants, glidants etc. Additives such as, e.g., pH adjusting agents, buffering agents, enhancers, wetting agents, solubilizing agents, surfactants, anti-oxidants etc. The term "loadable tablet" used in the present context denotes an "inert tablet" as defined above, but further having a porosity of at least about 45% v/v in order to enable a suitable loading with a liquid, a suitable hardness of at least 20kN that ensures that the tablet has sufficient mechanical strength to withstand normal handling, and moreover it has a loading capacity of 30% or more. However, in some cases it may be of interest to include an active substance in such a tablet and, accordingly, the term "loadable tablet" also includes such cases. In a preferred embodiment, the tablets are "inert and loadable", i.e. without any content of active substance before loading.

However, as shown in the Examples herein, the present inventors have found that it is possible to load tablets having a high porosity with a pharmaceutically acceptable liquid, preferably containing one or more therapeutically, prophylactically and/or diagnostically active substances (in the following abbreviated "active substance"). The loaded tablets are sufficiently robust to withstand the normal handling of tablets during further processing (e.g. coating), packaging, storage etc., i.e. they fulfill the pharmacopoeial requirements with respect to hardness and friability.

In a specific embodiment a disintegrating loadable tablet according to the invention results - when tested as described herein - in a loading of the tablet with at least 30% w/w such as, e.g., at least 35% w/w, at least 40% w/w, at least 45% w/w or at least 50% w/w of corn oil (based on the total weight of the solid dosage form upon loading). Such a test ensures that the tablet has the ability of sorbing a liquid formulation that is suitable for use in the preparation of tablets. An important aspect is also that once the liquid has been sorbed, it will remain in the tablet. Accordingly, the average weight of 20 tablets after loading with the liquid will at the most change $\pm$ 10% such as, e.g., at the most $\pm$ 7.5%, at the most $\pm$ 5%, at the most $\pm$ 2.5% or at the most $\pm$ 1% upon storage at normal temperature for at least 1 months such as, e.g., for at least 3 months, at least 6 months or at least 1 year.

As mentioned above, the loadable tablets according to the invention are sufficiently robust to withstand the normal handling of tablets, i.e. they have a hardness of 20 N or more such as, e.g., about 25 N or more, about 30 N or more, about 35 N or more, about 40 N or more, about 45 N or more or about 50 N or more. An upper limit for the hardness is about 150 N such as, e.g., about 120 N. There is naturally a balance with on the one hand a large porosity of the tablet and on the other hand a suitably large hardness. Normally a harder tablet leads to a less porous tablet, as the hardness is an expression of the compactability of the tablet, i.e. how firm the tablet has been compressed. However, by use of the sorbent materials described herein the inventors have found it possible to obtain a proper balance between sufficient hard tablets, excellent porosity of the tablets and excellent loading capacity of the tablets.

Furthermore, the tablets according to the invention have a friability of about 5% or less such as, e.g., about 4% or less, about 3% or less, about 2% or less such as about 1% or less, about 0.5% or less or about 0.1% or less.

As mentioned above, the loadable tablets according to the invention comprise one or more sorbent materials and, optionally further pharmaceutically acceptable excipients. It is however, important that a least one sorbent material has the right properties with respect to providing a tablet with a porosity of 45% v/v or more and that this sorbent material is present in a sufficient amount so that the tablet obtained also has the desired porosity. Such sorbent materials are generally pharmaceutically acceptable excipients and are in some cases herein denoted "pharmaceutically acceptable, porosity providing excipients". To this end, the present inventors have found that if the sorbent material is manufactured into tablets together with at the most 50% w/w of lactose or other pharmaceutically acceptable excipients used for direct compression such as, e.g., Emcompress, and the tablets obtained have a porosity of 45 vol % or more, then the pharmaceutically acceptable excipient is suitable for use in the present context. The quality of lactose is for direct compression.

In the loadable tablets the sum of sorbent materials that have the above-mentioned property (i.e. fulfils the above-mentioned test) corresponds to at least 50% w/w such as, e.g. at least 55% w/w, at least 60% w/w, at least 65% w/w, at least 70% w/w, at least 80% w/w, at least 90% w/w, at least 95% w/w or at least 98% w/w such as e.g. 100% w/w of the total weight of the tablet. As mentioned herein before, a disintegrant is also present in the tablet. Accordingly, in those cases where the disintegrant does not have the above-mentioned property relating to the porosity of the tablet, the sum of sorbent materials that have the above-mentioned property (i.e. fulfils the above-mentioned test) corresponds to at least 50% w/w such as, e.g. at least 55% w/w, at least 60% w/w, at least 65% w/w, at least 70% w/w, at least 80% w/w or at least 85% w/w and the concentration of the one or more disintegrants present in the tablet is at the most 15% w/w. To this end, it is important to note that it is an advantage to keep the concentrations of any other ingredients in the tablets as low as possible in order to ensure that the concentration of the sorbent material is so high as possible due to the fact that the loadability of the tablet is dependent on the porosity. In other words, if a high load is desired, then a high concentration of the porosity-providing excipients is preferred and *vice versa.*

In preferred aspects, the one or more sorbent materials are present in a concentration of about 50% w/w or more such as, e.g., about 60% w/w or more such as, e.g., about 70% w/w or more, about 80% w/w or more, about 90% w/w or more or about 95% w/w or more in the tablet.

Moreover, it is important that the specific surface area (BET surface area) of the sorbent material should be relatively large such as, e.g., at least 50 $m^2/g$ as measured by gas adsorption. In specific embodiments, the specific surface are is as large as about 100 $m^2/g$ or more such as, e.g., 150 $m^2/g$ or more, 200 $m^2/g$ or more, 250 $m^2/g$ or more, 300 $m^2/g$ or more, 350 $m^2/g$ or more or 400 $m^27g$ or more.

In the following is given a list of sorbent materials that are pharmaceutically acceptable material that have suitable properties that enable providing a suitable porosity of a disintegrating loadable tablet according to the invention. The individual sorbent materials may be used alone or in combination provided that the overall aim is obtained with respect to porosity.

To this end, it should be noted that the tablets are compressed into tablets by use of a certain compression force. However, the compression force may not be so low that the requirements with respect to hardness and friability of the tablets are compromised, i.e. these requirements ensure that the tablets are sufficiently robust.

Suitable sorbent materials that can be used to obtain tablets according to the invention are selected from the group consisting of metal oxides, metal silicates, metal carbonates, metal phosphates, metal sulfates, sugar alcohols, sugars and cellulose and cellulose derivatives. The metal is typically selected from the group consisting of sodium, potassium, magnesium, calcium, zink, aluminium, titanium and silicium.

A suitable metal oxide for use according to the invention may be selected from the group consisting of magnesium oxide, calcium oxide, zink oxide, aluminium oxide, titanium dioxide including Tronox A-HP-328 and Tronox A-HP-100, silicium dioxides including Aerosil, Cab-O-Sil, Syloid, Aeroperl, Sunsil (silicon beads), Zeofree, Sipernat, and mixtures thereof.

In a specific embodiment, the metal oxide is a titanium dioxide or a silicium dioxide or mixtures thereof.

The silicates can be divided in the following groups:

- Swelling clays of the smectite type e.g. bentonite, veegum, laponite.
- Hydrous aluminium silicates or alkaline earths. Neusilin belongs to this group and is based on synthetic polymerisation (magnesium aluminium metasilicate).

- Silicon dioxides are subdivided into porous and nonporous silicas

  o Nonporous colloidal silicas e.g. Aerosil (fumed silicas)
  o Porous silicas gels e.g. Syloid, Porasil, Lichrosorp
  o Others e.g. Zeopharm S170, Zeopharm 6000, Aeroperl 300

Neusilin is the trade name for different grades of magnesium aluminametasilicate with a Al2O3 content from 29.1 to 35.5%, a MgO content in the range of 11.4 to 14.0 % and a content of SiO2 from 29.2 to 35.6 percentage.

The preferred type of Neusilin according to the present invention is in a spherical fine granulate form which is suitable for tabletting and is referred to as the grades Neusilin S1 having a surface area of 110 $m^2$/g and a oil adsorbing capacity of 1.3 ml/g; Neusilin SG1 also having a surface area of 110 m2/g and a oil adsorbing capacity of 1.3 ml/g; Neusilin NS2N having a surface area of 250 $m^2$/g and a oil adsorbing capacity of 2.2 ml/g; and Neusilin US2 having a surface area of 300 $m^2$/g and a oil adsorbing capacity of 3.0 ml/g (according to the manufacture's specification).

Accordingly, a disintegrating loadable tablet according to the invention may contain a metal oxide that is a non-porous silicate including fumed silicas of the Aerosil type, and/or a porous silicate including e.g. Syloid, Porasil and Lichrosorp.

In other embodiments the sorbent material for use according to the invention is a metal silicate selected from the group consisting of sodium silicate, potassium silicate, magnesium silicate, calcium silicate including synthetic calcium silicate such as, e.g., Hubersorp, zink silicate, aluminum silicate, sodium aluminosilicate such as, e.g., Zeolex, magnesium aluminum silicate, magnesium aluminum metasilicate, aluminium metasilicate, Neusilin SG2 and Neusilin US2 and mixtures thereof.

The metal silicate may also be a swelling clay of the smectite type selected from the group consisting of bentonite, veegum and laponite, and/or the metal silicate is selected from alkaline earth metal silicates and aluminum silicates included magnesium aluminum metasilicate. In a specific embodiment the metal silicate is Neusilin.

Other sorbent materials may be found among the following excipients, although in some cases only specific qualities of the individual excipients may fulfill the requirement given above.

As mentioned above a suitable sorbent material may be a metal carbonate such as a carbonate selected from the group consisting of sodium carbonate, sodium hydrogen carbonate, potassium carbonate, potassium hydrogen carbonate, calcium carbonate, magnesium carbonate, zinc carbonate and aluminum carbonate, and mixtures thereof. Some of the mentioned carbonates may be especially suitable for use in the effervescent tablet disintegration formulation principle mentioned below.

Other metal salt suitable for use according to the invention are metal phosphates selected from the group consisting of sodium phosphate, disodium hydrogen phosphate, sodium dihydrogen phosphate, potassium phosphate, dipotassium hydrogen phosphate, potassium dihydrogen phosphate, calcium phosphate, magnesium phosphate, zink phosphate and aluminum phosphate.

More specifically, the sorbent material may be a calcium phosphate selected from the group consisting of dibasic anhydrous calcium phosphate, dibasic dihydrate calcium phosphate, and tribasic calcium phosphate.

The dibasic anhydrous calcium phosphate is typically selected from the group consisting of A-Tab, calcium monohydrogen phosphate, calcium orthophosphate, Di-Cafos AN, dicalcium orthophosphate, E341, Anhydrous Emcompress, Fujicalin, phosphoric acid calcium salt (1:1), and secondary calcium phosphate, and mixtures thereof. The dibasic dihydrate calcium phosphate may be selected from the group consisting of Cafos, calcium hydrogen orthophosphate dihydrate, calcium monohydrogen phosphate dihydrate, Calipharm, Calstar, Di-Cafos, dicalcium orthophosphate, DI-TAB, Emcompress, phosphoric acid calcium salt (1:1) dihydrate, secondary calcium phosphate, Fujiclin SG.

Examples of tribasic calcium phosphates are e.g. hydroxyapatite, phosphoric acid calcium salt (2:3), precipitated calcium phosphate, tertiary calcium phosphate, Tri-Cafos, tricalcium diorthophosphate, tricalcium orthophosphate, tricalcium phosphate, TRI-CAL, WG, TRI-TAB.

Other suitable metal salts are metal sulfates such as, e.g, sodium sulfate, sodium hydrogen sulfate, potassium sulfate, potassium hydrogen sulfate, calcium sulfate, magnesium sulfate, zink sulfate and/or aluminum sulfate.

Examples of suitable calcium sulfates are e.g. calcium sulfate anhydrous including anhydrite, anhydrous gypsum, anhydrous sulfate of lime, Destab, Drierte, E516, karstenite, muriacite, and Snow White or calcium sulfate dihydrate including alabaster, Cal-Tab, Compactrol, Destab, E516, gypsum, light spar, mineral white, native calcium sulfate, precipitated calcium sulfate, satinite, satin spar, selenite, terra alba and USG Terra Alba.

In other embodiments, the sorbent material may be a sugar alcohol selected from the group consisting of sorbitol (such as, e.g., Sorbogem, SPI Pharma), xylitol, mannitol (such as, e.g., Mannogem, SPI Pharma), maltitol, inositol, mannitol (e.g. Pealitol SP 100) and/or it may be a sugar selected from the group consisting of mono-, di- or polysaccharides including saccharose, glucose, fructose, sorbose, xylose, lactose, dextran, dextran derivatives, cyclodextrins. As appears from the following, such substances may also be included as a hydrophilic substance in order to act as a wetting agent or a humectant.

Cellulose and cellulose derivatives may also be suitable sorbent material for the purpose of obtaining tablets according to the invention. However, these substances seem to be less effective for providing sufficient porosity. Examples include cellulose, microcrystalline cellulose, Celphere, cellulose derivatives including porous cellulose beads: cellulose acetate Celluflow TA-25 and cellulose Celluflow C-25, hydroxypropyl methylcellulose (HPMC), hydroxypropyl cellulose (HPC), methylcellulose, ethylcellulose, sodium carboxymethylcellulose, hydroxyethyl cellulose etc.

As mentioned above, an important ingredient in a disintegrating porous tablet according to the invention is one or more disintegrants. To this end, both traditionally employed disintegrants as well as the so-called superdisintegrants can be employed. In a preferred embodiment it is desirable that the concentration of the disintegrant is as low as possible which gives an indication that a superdisintegrant is preferred provided that a sufficient decrease in disintegration time can be obtained (compared to a tablet formulated without any disintegrant). A superdisintegrant normally has more disintegrating power, i.e. an acceptable disintegration of a tablet can be obtained using low concentrations (i.e. below about 10-15% w/w) of the superdisintegrant.

Superdisintegrants are used in pharmaceutical solid dosage forms: tablets, granules, capsules or suppositories which when contact with gastrointestinal fluid will normally effectively increase disintegration. Their mechanisms of action include swelling, capillary action or deformation. General superdisintegrants, sodium carboxymethyl cellulose (Ac-Di-Sol®, CLD-2®) and Crosslinked polyvinylpyrrolidone (Polyplasdone-X1R, Polyplasdone-XL 10R, Kollidon-CLR) are obtained from various source and have different properties. At the low concentration of 1-8 percent, 1-3 percent and 0.5-5 percent respectively, they generally produce good disintegration. Their disintegrating efficiency depends on their own physical and chemical properties, diluent and process of preparation and storage.

Although there are many disintegrants for selection, searching for suitable disintegrants are ongoing and include using or modifying natural products, for example: formalin-casein, L-HPC, chitin, chitosan, polymerized agar acrylamide, xylan, smecta, key-jo-clay, crosslinked carboxymethylguar and modified tapioca starch which are all included for use as an disintegrant or component of a disintegrant mixture or the "disintegration principle" of the loaded tablet according to the present invention. Other examples include e.g. alginic acid or alginates, microcrystalline cellulose, hydroxypropyl cellulose and other cellulose derivatives, croscarmellose sodium, crospovidone, polacrillin potassium, sodium starch glycolate, starch, pregelatinized starch, carboxymethyl starch (e.g. Primogel® and Explotab®) etc.

With "disintegration principle" as used herein is meant a combination of substances which together improves the disintegration of the loaded tablet and includes for instance a swellable disintegrant together with an osmotic substance; a disintegrant together with an effervescent; and combination of different disintegrants. In addition, the term can be used for a selection of a specific disintegrant in relation to the carrier system as different carries have different effect on the disintegration of the tablet as also demonstrated in the Examples. Furthermore, a different disintegration principle might be applied for instance based on use of hitherto unknown excipients having disintegrating effect in the tablets according to the invention and/or combining disintegrants with other excipients having a synergistic or improving effect on disintegration and/or optimizing the disintegrant in relation to the carrier system and desired disintegration time or release. To this end, it should be mentioned that the present inventors have found that gellan gum in the present context behaves as a superdisintegrant. In a particular embodiment, the disintegrant is gellan gum or gellan gum is a component of a mixture of disintegrants.

Normally, the one or more disintegrant is present in a tablet according to the invention in a concentration of from 0.1 % w/w to 15 % w/w, such as of 0.2% to 10% w/w, such as 0.3 to 8% w/w, such as 0.4 to 8% w/w such as 0.5 to 5% w/w. In specific embodiments the concentration is at least 1% w/w.

More specifically, a tablet according to the invention comprising a disintegrant or a mixture of disintegrants decreases the disintegration time for the tablet compared with a tablet not comprising same amount of said disintegrant or mixture of disintegrants.

In a specific embodiment, a tablet according to the invention has a disintegration time of at the most 120 sec as tested according to Ph.Eur such as the most 90 sec, such as the most 60 sec, such as the most 45 sec, such as at the most 35 sec, such as the most 30, such at the most 25 sec.

Moreover or alternatively, in a specific embodiment, at tablet according to the invention the time for the release of 25% or 50% or 75% or 80% of the therapeutically, prophylactically and/or diagnostically active substance is decreased to at the most 50% such as at the most 40% such as at the most 30% such as the most 20% of the time for same % of release measured by dissolution of a similar tablet not comprising a disintegrant and measured in a dissolution method according to USP.

The disintegration and or release of the therapeutically, prophylactically and/or diagnostically active substance from a tablet of the invention may also be controlled.

To this end, at least 25% such as at least 33%, such as at least 50% w/w of the therapeutically, prophylactically and/or diagnostically active substance is released within 30 min when tested in a dissolution method according to USP, and/or at least 80% w/w of the therapeutically, prophylactically and/or diagnostically active substance is released within 45 min when tested in a dissolution method according to USP.

Moreover, in an interesting embodiment a disintegrant is used in combination with a hydrophilic substance that behaves like a wetting agent or a humectant, i.e. by facilitating contact between water and the solid tablet or tablet ingredients. It seems likely that such a substance contributes to the overall decrease in disintegration time (cf. the examples herein) and that the effect of the disintegrant(s) (including the superdisintegrants) is improved in the presence of such a hydrophilic substance. If present, the concentration of such a hydrophilic substance normally is at the most about 15% w/w such as, e.g. at the most about 10% w/w, at the most about 7.5% w/w, at the most about 5% w/w or at the most about 2.5% w/w.

Moreover, the hydrophilic substance may increase water contact with the disintegrant.

Examples of hydrophilic substances suitable for use in this context are e.g. sugars and sugar alcohols and polyols. Specific examples of suitable sugars include glucose, fructose, sucrose, maltose, xylose, sorbose, maltitose, raffinose and lactose.

Specific examples of suitable sugar alcohols include xylitol, erythitol, sorbitol, mannnitol, maltitol, inositiol, and specific examples of suitable polyols include e.g. glycerol.

Accordingly, the mixture of disintegrant mixture or principle according to the present invention may comprise two or more components selected from swellable gums, preferable gellan gum, superdisintegrants, effervescent and osmotic and/or hydrophilic components such as xylitol and other pharmaceutical excipients improving or controlling the disintegration properties of the tablet.

**Other pharmaceutically acceptable excipients for use in a disintegrating loadable tablet according to the invention**

The loadable tablet may of course also contain other pharmaceutically acceptable excipients such as those normally employed in the manufacturing of tablets.

In the present context the terms "pharmaceutically acceptable excipient" are intended to denote any material, which is inert in the sense that it substantially does not have any therapeutic and/or prophylactic effect *per se.* Such an excipient may be added with the purpose of making it possible to obtain a pharmaceutical, cosmetic and/or foodstuff composition, which have acceptable technical properties.

Examples of suitable excipients for use in A disintegrating loadable tablet according to the invention include fillers, diluents, disintegrants, binders, lubricants etc. or mixture thereof. As the composition or solid dosage form according to the invention may be used for different purposes, the choice of excipients is normally made taken such different uses into considerations. Other pharmaceutically acceptable excipients for suitable use are e.g. acidifying agents, alkalizing agents, preservatives, antioxidants, buffering agents, chelating agents, coloring agents, complexing agents, emulsifying and/or solubilizing agents, flavors and perfumes, humectants, sweetening agents, wetting agents etc.

Examples of suitable fillers, diluents and/or binders include lactose (e.g. spray-dried lactose, $\alpha$-lactose, $\beta$-lactose, Tabletose®, various grades of Pharmatose®, Microtose® or Fast-Floc®), microcrystalline cellulose (various grades of Avicel®, Elcema®, Vivacel®, Ming Tai® or Solka-Floc®), hydroxypropylcellulose, L-hydroxypropylcellulose (low substituted), hydroxypropyl methylcellulose (HPMC) (e.g. Methocel E, F and K, Metolose SH of Shin-Etsu, Ltd, such as, e.g. the 4,000 cps grades of Methocel E and Metolose 60 SH, the 4,000 cps grades of Methocel F and Metolose 65 SH, the 4,000, 15,000 and 100,000 cps grades of Methocel K; and the 4,000, 15,000, 39,000 and 100,000 grades of Metolose 90 SH), methylcellulose polymers (such as, e.g., Methocel A, Methocel A4C, Methocel A15C, Methocel A4M), hydroxyethylcellulose, sodium carboxymethylcellulose, carboxymethylene, carboxymethylhydroxyethylcellulose and other cellulose derivatives, sucrose, agarose, sorbitol, mannitol, dextrins, maltodextrins, starches or modified starches (including potato starch, maize starch and rice starch), calcium phosphate (e.g. basic calcium phosphate, calcium hydrogen phosphate, dicalcium phosphate hydrate), calcium sulfate, calcium carbonate, sodium alginate, collagen etc.

Specific examples of diluents are e.g. calcium carbonate, dibasic calcium phosphate, tribasic calcium phosphate, calcium sulfate, microcrystalline cellulose, powdered cellulose, dextrans, dextrin, dextrose, fructose, kaolin, lactose, mannitol, sorbitol, starch, pregelatinized starch, sucrose, sugar etc.

Specific examples of binders are e.g. acacia, alginic acid, agar, calcium carrageenan, sodium carboxymethylcellulose, microcrystalline cellulose, dextrin, ethylcellulose, gelatin, liquid glucose, guar gum, hydroxypropyl methylcellulose, methylcellulose, pectin, PEG, povidone, pregelatinized starch etc.

Glidants and lubricants may also be included in the tablet. Examples include stearic acid, magnesium stearate, calcium stearate or other metallic stearate, talc, waxes and glycerides, light mineral oil, PEG, glyceryl behenate, colloidal silica, hydrogenated vegetable oils, corn starch, sodium stearyl fumarate, polyethylene glycols, alkyl sulfates, sodium benzoate, sodium acetate etc.

Other excipients which may be included in A disintegrating loadable tablet of the invention are e.g. flavoring agents, coloring agents, taste-masking agents, pH-adjusting agents, buffering agents, preservatives, stabilizing agents, anti-oxidants, wetting agents, humidity-adjusting agents, surface-active agents, suspending agents, absorption enhancing agents, agents for modified release etc.

Other additives in a composition or a solid dosage form according to the invention may be antioxidants like e.g. ascorbic acid, ascorbyl palmitate, butylated hydroxyanisole, butylated hydroxytoluene, hypophosphorous acid, monothioglycerol, potassium metabisulfite, propyl gallate, sodium formaldehylde sulfoxylate, sodium metabisulfite, sodium thiosulfate, sulfur dioxide, tocopherol, tocopherol acetate, tocopherol hemisuccinate, TPGS or other tocopherol derivatives, etc. The carrier composition may also contain e.g. stabilising agents. The concentration of an antioxidant and/or a stabilizing agent in the carrier composition is normally from about 0.1 % w/w to about 5% w/w.

A composition or solid dosage form according to the invention may also include one or more surfactants or substances having surface-active properties. It is contemplated that such substances are involved in the wetting of the slightly soluble active substance and thus, contributes to improved solubility characteristics of the active substance.

Examples on surfactants are given in the following.

Suitable excipients for use in a tablet according to the invention are surfactants such as, e.g., amphiphillic surfactants as those disclosed in WO 00/50007 in the name of Lipocine, Inc. Examples on suitable surfactants are

i) polyethoxylated fatty acids such as, e.g. fatty acid mono- or diesters of polyethylene glycol or mixtures thereof such as, e.g. mono - or diesters of polyethylene glycol with lauric acid, oleic acid, stearic acid, myristic acid, ricinoleic acid, and the polyethylene glycol may be selected from PEG 4, PEG 5, PEG 6, PEG 7, PEG 8, PEG 9, PEG 10, PEG 12, PEG 15, PEG 20, PEG 25, PEG 30, PEG 32, PEG 40, PEG 45, PEG 50, PEG 55, PEG 100, PEG 200, PEG 400, PEG 600, PEG 800, PEG 1000, PEG 2000, PEG 3000, PEG 4000, PEG 5000, PEG 6000, PEG 7000, PEG 8000, PEG 9000, PEG 1000, PEG 10,000, PEG 15,000, PEG 20,000, PEG 35,000,

ii) polyethylene glycol glycerol fatty acid esters, i.e. esters like the above-mentioned but in the form of glyceryl esters of the individual fatty acids;

iii) glycerol, propylene glycol, ethylene glycol, PEG or sorbitol esters with e.g. vegetable oils like e.g. hydrogenated castor oil, almond oil, palm kernel oil, castor oil, apricot kernel oil, olive oil, peanut oil, hydrogenated palm kernel oil and the like,

iv) polyglycerized fatty acids like e.g. polyglycerol stearate, polyglycerol oleate, polyglycerol ricinoleate, polyglycerol linoleate,

v) propylene glycol fatty acid esters such as, e.g. propylene glycol monolaurate, propylene glycol ricinoleate and the like,

vi) mono- and diglycerides like e.g. glyceryl monooleate, glyceryl dioleae, glyceryl mono- and/or dioleate, glyceryl caprylate, glyceryl caprate etc.;

vii) sterol and sterol derivatives;

viii) polyethylene glycol sorbitan fatty acid esters (PEG-sorbitan fatty acid esters) such as esters of PEG with the various molecular weights indicated above, and the various Tween ® series;

ix) polyethylene glycol alkyl ethers such as, e.g. PEG oleyl ether and PEG lauryl ether;

x) sugar esters like e.g. sucrose monopalmitate and sucrose monolaurate;

xi) polyethylene glycol alkyl phenols like e.g. the Triton® X or N series;

xii) polyoxyethylene-polyoxypropylene block copolymers such as, e.g., the Pluronic® series, the Synperonic® series, Emkalyx®, Lutrol®, Supronic® etc. The generic term for these polymers is "poloxamers" and relevant examples in the present context are Poloxamer 105, 108, 122, 123, 124, 181, 182, 183, 184, 185, 188, 212, 215, 217, 231, 234, 235, 237, 238, 282, 284, 288, 331, 333, 334, 335, 338, 401, 402, 403 and 407;

xiii) sorbitan fatty acid esters like the Span® series or Ariacel® series such as, e.g. sorbinan monolaurate, sorbitan monopalmitate, sorbitan monooleate, sorbitan monostearate etc.;

xiv) lower alcohol fatty acid esters like e.g. oleate, isopropyl myristate, isopropyl palmitate etc.;

xv) ionic surfactants including cationic, anionic and zwitterionic surfactants such as, e.g. fatty acid salts, bile salts, phospholipids, phosphoric acid esters, carboxylates, sulfates and sulfonates etc.

When a surfactant or a mixture of surfactants is present in a composition or a solid dosage form of the invention, the concentration of the surfactant(s) is normally in a range of from about 0.1 - 80% w/w such as, e.g., from about 0.1 to about 20% w/w, from about 0.1 to about 15% w/w, from about 0.5 to about 10% w/w, or alternatively, from about 0.10 to about 80% w/w such as, e.g. from about 10 to about 70% w/w, from about 20 to about 60% w/w or from about 30 to about 50% w/w.

**Tablets loaded with a pharmaceutically acceptable liquid**

The tablets described above are designed so that they can be loaded with pharmaceutically acceptable liquid formulation in a concentration of about 20% w/w or more such as, e.g., about 25% w/w or more, about 30% w/w or more (based on the total weight of the solid dosage form upon loading). Accordingly, in another aspect the invention relates to such tablets.

The blank tablets (i.e. the loadable tablets) can be loaded by immersing the tablets in a liquid containing the active substance(s). As described in the examples herein, the tablets will soak a well-defined amount of the liquid, i.e. they will be loaded with a well-defined amount of the active substance. Other methods may also be employed such as spraying the liquid on the tablets in a suitable apparatus. There is no need to remove an excess of liquid from the tablets, e.g. by centrifugation; after drying in the air, the tablets appear dry and smooth without any sticky appearance.

In preferred aspects, the pharmaceutically acceptable liquid formulation is present in a concentration of about 40% w/w or more such as, e.g., about 50% w/w or more or about 60% w/w or more (based on the total weight of the solid dosage form upon loading).

A critical parameter in connection with the loading of the liquid formulation is the viscosity of the liquid formulation. The loading can be performed in any possible manner such as, e.g., by placing the tablets in a suitable container containing the liquid or by spraying the liquid on the tablets in a suitable apparatus such as, e.g., using conventional coating equipment such as coating pan, perforated vessel or fluidized bed. Especially the viscosity of the liquid is important when the liquid formulation is sprayed on the tablets. Accordingly, in a specific embodiment the pharmaceutically acceptable liquid formulation has a viscosity of at the most about 600 mPa sec at a temperature of at the most about 150 °C.

Furthermore, the pharmaceutically acceptable liquid formulation normally has a melting point of at least about 0 °C and at the most about 250 °C such as, e.g., about 5 °C or more such as, e.g., about 10 °C or more, about 15 °C or more, about 20 °C or more or about 25 °C or more. The melting point is not very critical as the liquid formulation may be heated or cooled in connection with loading of the tablets with the liquid formulation.

The pharmaceutically acceptable liquid formulation can be based on water or it can be based on an organic solvent or an oil or an oily-like material. Surprisingly, the inventors have found that A disintegrating loadable tablet according to the invention can be dipped into water and upon saturation with water (which takes only a few minutes or less) the tablet appear with a cold, but dry surface, i.e. water and aqueous based liquid can also be employed a suitable pharmaceutically acceptable liquid formulation.

However, the more general applicability is envisaged with respect to loading the tablets with active substances contained in an aqueous or organic based liquid. Such liquids include oil or oily-like materials or pharmaceutically acceptable solvents.

Such oils or oily-like materials may be selected from the group consisting of water, vegetable oils, hydrogenated vegetable oils, and animal oils.

Suitable examples include apricot oil, almond oil, avocado oil, castor oil, coconut fat, cocoa butter, corn oil, cotton seed oil, grape seed oil, jojoba oil, linseed oil, maize oil, olive oil, palm oil, peanut oil, persil oil, poppy seed oil, rape seed oil, sesame oil, soybeen oil, sunflower oil, thistle seed oil, walnut oil, wheat germ oil, beef tallow, lard, tall oil, whale oil, and mixtures thereof.

Other examples are hydrophilic oils or oily-like materials selected from the group consisting of: polyether glycols such as, e.g., polyethylene glycols, polypropylene glycols; polyoxyethylenes; polyoxypropylenes; poloxamers and mixtures thereof, or it may be selected from the group consisting of: xylitol, sorbitol, potassium sodium tartrate, sucrose tribehenate, glucose, rhamnose, lactitol, behenic acid, hydroquinon monomethyl ether, sodium acetate, ethyl fumarate, myristic acid, citric acid, Gelucire 50/13, other Gelucire types such as, e.g., Gelucire 44/14 etc., Gelucire 50/10, Gelucire 62/05, Sucro-ester 7, Sucro-ester 11, Sucro-ester 15, maltose, mannitol and mixtures thereof.

The oil or oily-like material may also be a hydrophobic oil or oily-like material selected from the group consisting of: straight chain saturated hydrocarbons, sorbitan esters, paraffins; fats and oils such as e.g., cacao butter, beef tallow, lard, polyether glycol esters; higher fatty acid such as, e.g., stearic acid, myristic acid, palmitic acid, higher alcohols such as, e.g., cetanol, stearyl alcohol, low melting point waxes such as, e.g., glyceryl monostearate, glyceryl monooleate, hydrogenated tallow, myristyl alcohol, stearyl alcohol, substituted and/or unsubstituted monoglycerides, substituted and/or unsubstituted diglycerides, substituted and/or unsubstituted triglycerides, yellow beeswax, white beeswax, carnauba wax, castor wax, japan wax, acetylate monoglycerides; NVP polymers, PVP polymers, acrylic polymers, or a mixture thereof.

Suitable polyethylene glycols generally have an average molecular weight in a range of from about 400 to about 35,000 such as, e.g., from about 800 to about 35,000, from about 1,000 to about 35,000 such as, e.g., polyethylene glycol 1,000, polyethylene glycol 2,000, polyethylene glycol 3,000, polyethylene glycol 4,000, polyethylene glycol 5,000, polyethylene glycol 6000, polyethylene glycol 7,000, polyethylene glycol 8,000, polyethylene glycol 9,000 polyethylene glycol 10,000, polyethylene glycol 15,000, polyethylene glycol 20,000, or polyethylene glycol 35,000. In certain situations polyethylene glycol may be employed with a molecular weight from about 35,000 to about 100,000.

In a specific embodiment, the oil or oily-like material may be a polyethylene oxide having a molecular weight of from about 2,000 to about 7,000,000 such as, e.g. from about 2,000 to about 100,000, from about 5,000 to about 75,000, from about 10,000 to about 60,000, from about 15,000 to about 50,000, from about 20,000 to about 40,000, from about 100,000 to about 7,000,000 such as, e.g., from about 100,000 to about 1,000,000, from about 100,000 to about 600,000, from about 100,000 to about 400,000 or from about 100,000 to about 300,000.

Poloxamers can also be used according to the invention. Examples include Poloxamer 188, Poloxamer 237, Poloxamer 338 or Poloxamer 407 or other block copolymers of ethylene oxide and propylene oxide such as the Pluronic® and/or Tetronic® series. Suitable block copolymers of the Pluronic® series include polymers having a molecular weight of about 3,000 or more such as, e.g. from about 4,000 to about 20,000 and/or a viscosity (Brookfield) from about 200 to about 4,000 cps such as, e.g., from about 250 to about 3,000 cps. Suitable examples include Pluronic® F38, P65, P68LF, P75, P77, P84, P86, F87, F88, F98, P103, P104, P105, F108, P123, F123, F127, 10R8, 17R8, 25R5, 25R8 etc. Suitable block copolymers of the Tetronic® series include polymers having a molecular weight of about 8,000 or more such as, e.g., from about 9,000 to about 35,000 and/or a viscosity (Brookfield) of from about 500 to about 45,000 cps such as, e.g., from about 600 to about 40,000. The viscosities given above are determined at 60 °C for substances that are pastes at room temperature and at 77 °C for substances that are solids at room temperature.

In another embodiment, the oil or oily-like material may be a sorbitan ester such as, e.g., sorbitan di-isostearate, sorbitan dioleate, sorbitan monolaurate, sorbitan monoisostearate, sorbitan monooleate, sorbitan monopalmitate, sorbitan monostearate, sorbitan sesqui-isostearate, sorbitan sesquioleate, sorbitan sesquistearate, sorbitan tri-isostearate, sorbitan trioleate, sorbitan tristearate or mixtures thereof.

Moreover or alternatively, the oil or oily-like material may be a mixture of different oils or oily-like materials such as, e.g., a mixture of hydrophilic and/or hydrophobic materials, or a solvent or a semi-solid excipient like, e.g. propylene glycol, polyglycolised glycerides including Gelucire 44/14, complex fatty materials of plant origin including theobroma oil, carnauba wax, vegetable oils like e.g. almond oil, coconut oil, corn oil, cottonseed oil, sesame oil, soya oil, olive oil, castor oil, palm kernels oil, peanut oil, rape oil, grape seed oil etc., hydrogenated vegetable oils such as, e.g. hydrogenated peanut oil, hydrogenated palm kernels oil, hydrogenated cottonseed oil, hydrogenated soya oil, hydrogenated castor oil, hydrogenated coconut oil; natural fatty materials of animal origin including beeswax, lanolin, fatty alcohols including cetyl, stearyl, lauric, myristic, palmitic, stearic fatty alcohols; esters including glycerol stearate, glycol stearate, ethyl oleate, isopropyl myristate; liquid interesterified semi-synthetic glycerides including Miglycol 810/812; amide or fatty acid alcolamides including stearamide ethanol, diethanolamide of fatty coconut acids, acetic acid esters of mono and di-glycerides, citric acid esters of mono and di-glycerides, lactic acid esters of mono and diglycerides, mono and di-glycerides, poly-glycerol esters of fatty acids, poly-glycerol poly-ricinoleate, propylene glycol esters of fatty acids, sorbitan monostearates, sorbitan tristearates, sodium stearoyl lactylates, calcium stearoyl lactylates, diacetyl tartaric acid esters of mono and di-glycerides etc.

The pharmaceutically acceptable liquid formulation may also be a dispersion including an emulsion, a microemulsion e.g. a self-microemulsifying drug delivery system (SMEDDS) or a suspension.

Typically the concentration of the pharmaceutically acceptable liquid formulation in the tablet is about 5% w/w or more such as, e.g., about 10% w/w or more, about 15% w/w or more, about 20% w/w or more, about 25% w/w or more, about 30% w/w or more, about 35% w/w or more, about 40% w/w or more, about 45% w/w or more, about 50 w/w or more, about 60% w/w or more or about 70% or more.

The tablets obtained after loading of a disintegrating loadable tablet with a pharmaceutically acceptable liquid formulation typically fulfill the pharmacopoeia requirements. Thus, a tablet according to the invention typically has a hardness of at least about 20 N and/or a friability of at the most about 5% such as, e.g., at the most about 4%, at the most about 3%, at the most about 2%, at the most about 1 % or at the most about 0.5%.

Furthermore, it is contemplated that the loading of the liquid into a disintegrating loadable tablet of the invention results in a substantially homogeneous distribution of the liquid within the tablet.

Furthermore, the tablets can be designed to release the active substance substantially immediately or in a modified manner. A tablet designed to immediate release typically has a disintegration time of at the most 15 min as tested according to Ph.Eur, whereas a film coated tablet may have a disintegration time of at the most about 30 min. For modified release tablets, the release of the active substance is of importance.

For a plain tablet according to the invention at least 75% of the therapeutically, prophylactically and/or diagnostically active substance is released within 30 min when tested in a dissolution method according to USP. In another embodiment the release is 80% within 45 minutes for the tablet.

As mentioned above, a preferred embodiment is a tablet loaded with one or more therapeutically, prophylactically and/or diagnostically active substances.

## Effervescent tablet disintegration formulation principle

In highly lipophilic environment the swellable properties of a disintegrant may be reduced and, accordingly, either a further disintegrant or excipient may be employed or another disintegration principle may be applied.

Such other excipients for use in combination with disintegrants may be substances having effervescent effect or highly osmotic or hydrophilic substances, which increase the amount of water coming into contact with the disintegrant. The latter may be of importance with a gelling agent as the gel over time may cause a dissolution layer having a relative low water dissolution rate. An effervescent may decrease the disintegration by the internal release of carbon dioxide. An effervescent tablet formulation is based on a combination of metal carbonates with and acid source. Metal carbonates are such as sodium bicarbonate, sodium carbonate, potassium bicarbonate, potassium carbonate, calcium carbonate, and sodium sesquicarbonate. The acid sources are such as citric acid, sodium dihydrogen citrate, disodium hydrogen citrate, tartaric acid, malic acid, fumaric acid, sodium dihydrogen phosphate, and sodium acid sulfite. The acid component might be excluded in the tablet formulation as the effervescent effect is obtained in-vivo when the tablet is dissolved in the acid gastric juice and reacts with the metal carbonate.

**Coating**

The tablet may also be coated with a film coating e.g. for immediate or modified release, an enteric coating, a modified release coating, a protective coating, an anti-adhesive coating etc.

Suitable coating materials are e.g. methylcellulose, hydroxypropylmethylcellulose, hydroxypropylcellulose, acrylic polymers, ethylcellulose, cellulose acetate phthalate, polyvinyl acetate phthalate, hydroxypropyl methylcellulose phthalate, polyvinylalcohol, sodium carboxymethylcellulose, cellulose acetate, cellulose acetate phthalate, gelatin, methacrylic acid copolymer, polyethylene glycol, shellac, sucrose, titanium dioxide, carnauba wax, microcrystalline wax, zein.

Plasticizers and other ingredients may be added in the coating material. The same or different active substance may also be added in the coating material.

*Melt coating*

The hydrophobic surface of a lipid loaded tablet according to the invention might prevent adhesion of a coating polymer applied in aqueous or organic solvent. As alternative, melt coating is suitable using different lipophilic meltable lipids sprayed in melted form and solidified onto tablet surface using conventional coating equipment. Useful melt coating substances are such as, polyglycolised glycerides (Gelucire 50/02, Gelucire 62/05, Gelucire 53/10), polyglyceryl palmitostearate, Glyceryl behenate (Compritol 888 ATO), glyceryl stearate (Precirol WL), glyceryl palmito stearate (Precirol ATO 5), polyglycolised unsaturated glycerides (Labrafil M 1944).

**Active substances**

In the present context a therapeutically and/or prophylactically active substance includes any biologically and/or physiologically active substance that has a function on an animal such as, e.g. a mammal like a human. The term includes drug substances, hormones, genes or gene sequences, antigen- comprising material, proteins, peptides, nutrients like e.g. vitamins, minerals, lipids and carbohydrates and mixtures thereof. Thus, the term includes substances that have utility in the treatment and/or preventing of diseases or disorders affecting animals or humans, or in the regulation of any animal or human physiological condition. The term also includes any biologically active substance which, when administered in an effective amount, has an effect on living cells or organisms.

Examples on active substances suitable for use in a tablet according to the invention are in principle any active substance such as, e.g. freely water soluble as well as more slightly or insoluble active substances. Thus, examples on active substances suitable for use are e.g. antibacterial substances, antihistamines and decongestants, anti-inflammatory agents, antiparasitics, antivirals, local anesthetics, antifungals, amoebicidals or trichomonocidal agents, analgesics, antianxiety agents, anticlotting agents, antiarthritics, antiasthmatics, antiarthritic, anticoagulants, anticonvulsants, antidepressants, antidiabetics, antiglaucoma agents, antimalarials, antimicrobials, antineoplastics, antiobesity agents, antipsychotics, antihypertensives, antitussives, auto-immune disorder agents, anti-impotence agents, anti-Parkinsonism agents, anti-Alzheimers' agents, antipyretics, anticholinergics, anti-ulcer agents, anorexic, beta-blockers, beta-2 agonists, beta agonists, blood glucose-lowering agents, bronchodilators, agents with effect on the central nervous system, cardiovascular agents, cognitive enhancers, contraceptives, cholesterol-reducing agents, cytostatics, diuretics, germicidals, H-2 blockers, hormonal agents, hypnotic agents, inotropics, muscle relaxants, muscle contractants, physic energizers, sedatives, sympathomimetics, vasodilators, vasoconstrictors, tranquilizers, electrolyte supplements, vitamins, counterirritants, stimulants, anti-hormones, drug antagonists, lipid-regulating agents, uricosurics, cardiac glycosides, expectorants, purgatives, contrast materials, radiopharmaceuticals, imaging agents, peptides, enzymes, growth factors, etc.

Specific examples include e.g.

Anti-inflammatory drugs like e.g. ibuprofen, indometacin, naproxen, nalophine;

Anti-Parkinsonism agents like e.g. bromocriptine, biperidin, benzhexol, benztropine etc.

Antidepressants like e.g. imipramine, nortriptyline, pritiptyline, etc.

Antibiotics like e.g. clindamycin, erythomycin, fusidic acid, gentamicin, mupirocine, amfomycin, neomycin, metronidazol, sulphamethizole, bacitracin, framycetin, polymyxin B, acitromycin etc,

Antifungal agents like e.g. miconazol, ketoconaxole, clotrimazole, amphotericin B, nystatin, mepyramin, econazol, fluconazol, flucytocine, griseofulvin, bifonazole, amorofine, mycostatin, itraconazole, terbenafine, terconazole, tolnaftate etc.

Antimicrobial agents like e.g.metronidazole, tetracyclines, oxytetracylines, peniciilins etc.

Antiemetics like e.g. metoclopramide, droperidol, haloperidol, promethazine etc.

Antihistamines like e.g. chlorpheniramine, terfenadine, triprolidine etc.

Antimigraine agents like e.g. dihydroergotamine, ergotamine, pizofylline etc.

Coronary, cerebral or peripheral vasodilators like e.g. nifedipine, diltiazem etc.

Antianginals such as, e.g., glyceryl nitrate, isosorbide dinitrate, molsidomine, verapamil etc.

Calcium channel blockers like e.g. verapamil, nifedipine, diltiazem, nicardipine etc.

Hormonal agents like e.g. estradiol, estron, estriol, polyestradiol, polyestriol, dienestrol, diethylstilbestrol, progesterone, dihydroprogesterone, cyprosterone, danazol, testosterone etc.

Contraceptive agents like e.g. ethinyl estradiol, lynestrenol, etynodiol, norethisterone, mestranol, norgestrel, levonorgestrel, desodestrel, medroxyprogesterone etc.

Antithrombotic agents like e.g. heparin, warfarin etc.

Diuretics like e.g. hydrochlorothiazide, flunarizine, minoxidil etc.

Antihypertensive agents like e.g. propanolol, metoprolol, clonidine, pindolol etc.

Corticosteroids like e.g. beclomethasone, betamethasone, betamethasone-17-valerate, betamethasone-dipropionate, clobetasol, clobetasol-17-butyrate, clobetasol-propionate, desonide, desoxymethasone, dexamethasone, diflucortolone, flumethasone, flumethasone-pivalte, fluocinolone acetonide, fluocinoide, hydrocortisone, hydrocortisone-17-butyrate, hydrocortisonebuteprate,methylprednisolone, triamcinolone acetonide, hacinonide, fluprednide acetate, alklometasone-dipropionate, fluocortolone, fluticason-propionte, mometasone-furate, desoxymethasone, diflurason-diacetate, halquinol, cliochinol, chlorchinaldol, fluocinolone-acetonide etc.

Dermatological agents like e.g. nitrofurantoin, dithranol, clioquinol, hydroxyquinoline, isotretinin, methoxsalen, methotrexate, tretinion, trioxalen, salicylic acid, penicillamine etc.

Steroids like e.g. estradiol, progesterone, norethindrone, levonorgestrel, ethynodiol, levonorgestrol, norgestimate, gestanin, desogestrel, 3-keton-desogesterel, demegestone, promethoestrol, testosterone, spironolactone and esters thereof etc.

Nitro compounds like e.g. amyl nitrates, nitroglycerine and isosorbide nitrate etc.

Opioids like e.g. morphine, buprenorphine, oxymorphone, hydromorphone, codeine, tramadol etc.

Prostaglandins such as, e.g., a member of the PGA, PGB, PGE or PGF series such as, e.g. minoprostol, dinoproston, carboprost, eneprostil etc.

Peptides like e.g. growth hormone releasing factors, growth factors (e.g. epidermal growth factor (EGF), nerve growth factor (NGF), TGF, PDGF, insulin growth factor (IGF), fibroblast growth factor (aFGF, bFGF etc.), somatostatin, calcitonin, insulin, vasopressin, interferons, IL-2 etc., urokinase, serratiopeptidase, superoxide dismutase, thyrotropin releasing hormone, lutenizing hormone releasing hormone (LH-RH), corticotrophin releasing hormone, growth hormone releasing hormone (GHRH), oxytocin, erythropoietin (EPO), colony stimulating factor (CSF) etc.

Other active substances of interest include ubiquinone (Coenzyme Q10), omega-3 fatty acids including fish oils containing such fatty acids, statins including simvastatin, lovastatin, atorvastatin, pravastatin, fluvastatin, rosuvastatin etc., fenofibrate.

Interesting examples are also prescription drugs like:

*Cardiovascular drugs*

Zocor®, Lipitor®, Prevachol®, Mevalotin®, Mevacor®, Lescol®, Tricor®, Norvasc®, Cozaar and Hyzaar®, Prinivil and Prinzide®, Diovan®/Co-Diovan®, Zestril®, Vasotech® and Vaseretic®, Lotensin®/Cibacen® and Lotrel®, Adalat®, Toprol-XL®/Seloken®, Tritace®/Delix®, Accupril® and Accuretic®, Avapro® and Avalide®, Plendil®, Monopril®, Blopress®, Atacand®, Tenormin®, Avapro®/Aprovel®, Coreg®, Altace®, Capoten®, Plavix®, Lovenox®/Clexane®, Fraxiparine®, ReoPro®, Panaldine®, Cordarone®

*Central nervous system drugs*

Paxil/Seroxat®, Zolotoft®, Prozac®, Prozac Weekly® and Sarafem®, Effexor®, Wellbutrin®, Celexa®, Remeron®, Serzone®, Zyprexa®, Risperdal®, Seroquel®, Clozaril®/Leponex®, Neurontin®, Depaktoke®, Lamictal®, Topamax®, Tegretol®, Imitrex®/Imigran®, Zomig®, Maxalt®, Ambien®, Stilnox®, Ultane®/Sevorane®, Diprivan®, BuSpar®, Xanax®, Aricept®, Memantine®, Adderall®, Dystonia®, Botox®

*Anti-infective agents*

Augmentin®, Cipro®/Ciprobay®, Zithromax®, Biaxin®, Levaquin® and Floxin®, Rocephin®, Primaxin®, Ceftin®/Zinnat®, Cravat®, Zosyn®/Tazocin©, Cefzil®, Sequin®, Tortaz®/Fortum®, Combivir®, Merit®, Valtrex®, Epivir®, Zovirax®, Crixivan®, Viracept®, Viramune®, Kaletra®, Diflucan®, Lamisil®, Sporanox®

*Respiratory drugs*

ClaritinAllegra®, Telfast®, Zyrtec®, Flonase®/Flixonase®, Atrovent®, Nasonex®, Rhinocort®, Alesion®, Singulair®, Flovent®/Flixotide®, Advair®/Seretide®, Serevent®, Pulmicort®, Ventoline®, Combivent®, Synagis®, Mucosolvan®

*Gastrointestinal drugs*

Prilosec®/Losec®, Prevacid®, Gasper®, Takepron®, Zantac®, Pantozol, Nexium, Protonix®, Aciphex®/Pariet®, Pepcid®, Axid®, Zoton®, Zofran®

*Cancer drugs*

Taxol®, Taxotere®, Nolvadex®, Herceptin, Ellence®/Pharmorubicin®, Lupron®, Zoladex®, Leuplin®, Casodex®, Intron A®, Peg-Intron® and Rebertron®, Rituxan®, Gemzar®, Paraplatin®, Camptosar®

*Antiarthritic drugs/analgesics*

Celebrex®, Vioxx®, Enbrel®, Remicade®, Voltaren®, Mobic®

Duragesic®

Ultram ®and Ultrcet®

*Blood disorder treatments*

Procrit®/Eprex®, Epogen®, Epogin®, NeoRecormon®, Neupogen®, NovoSeven®

*Diabetes drugs*

Glucophage®, Humulin Avandia®, Humalog®, Actos®, Amaryl®, Glucovance®, Glucophage XR®, Glucotrol XL®, Precose®/Glucobay®

*Bone metabolism regulators*

Fosamax®, Evista®, Miacalcin®, Actone®l, Aredia®

*Urinary disorder agents*

Harnal®, Proscar®, Cardura®, Flomax®, Detrol®

*Hormones*

Premarin®, Premphase® and prempro®, Estraderm®, Synthroid®

*Immunosuppressive agents*

Neoral®/Sandimmun®, CellCept, Rapamune®, Tacrolimus e.g. Program®, Medrol®

*Multiple Sclerosis drugs*

Avonex®, Betaseron®/Betaferon®, Rebif®, Copaxone®

*Biologicals*

Prevnar®, Engerix-B®, Infanrix®, Gamimune N®

*Sexual dysfunction drugs*

Viagra®

*Imaging agents*

Iopamiron®, Omnipaque®, Magnevist®

*Ophthalmic drugs*

Xalatan®, Trusopt® and Cosopt®

*Dermatological drugs*

Accutane®/Roaccutan®, Cleocin®

*Growth failure therapies*

Genotropin®, Humatrope®

*Infertility drugs*

Gonal-F®, Follistim(Puregon®

*Gaucher disease drugs*

Cerezyme®

*Obesity drugs*

Xencial®

*Acromegaly drugs*

Sandostatin®

*Contraceptives*

Depo-Provera®

Other interesting examples of active substances that are slightly soluble, sparingly soluble or insoluble in water are given in the following tables:

Table 1

| Poorly-Soluble Drug Candidates | | |
|---|---|---|
| **Drug Name** | **Therapeutic Class** | **Solubility In Water** |
| Alprazolam | CNS | Insoluble |
| Amiodarone | Cardiovascular | Very Slightly |
| Amlodipine | Cardiovascular | Slightly |
| Astemizole | Respiratory | Insoluble |
| Atenolol | Cardiovascular | Slightly |
| Azathioprine | Anticancer | Insoluble |
| Azelastine | Respiratory | Insoluble |

(continued)

| Poorly-Soluble Drug Candidates | | |
| --- | --- | --- |
| **Drug Name** | **Therapeutic Class** | **Solubility In Water** |
| Beclomethasone | Respiratory | Insoluble |
| Budesonide | Respiratory | Sparingly |
| Buprenorphine | CNS | Slightly |
| Butalbital | CNS | Insoluble |
| Carbamazepine | CNS | Insoluble |
| Carbidopa | CNS | Slightly |
| Cefotaxime | Anti-infective | Sparingly |
| Cephalexin | Anti-infective | Slightly |
| Cholestyramine | Cardiovascular | Insoluble |
| Ciprofloxacin | Anti-infective | Insoluble |
| Cisapride | Gastrointestinal | Insoluble |
| Cisplatin | Anticancer | Slightly |
| Clarithromycin | Anti-infective | Insoluble |
| Clonazepam | CNS | Slightly |
| Clozapine | CNS | Slightly |
| Cyclosporin | Immunosuppressant | Practically Insoluble |
| Diazepam | CNS | Slightly |
| Diclofenac sodium | NSAID | Sparingly |
| Digoxin | Cardiovascular | Insoluble |
| Dipyridamole | Cardiovascular | Slightly |
| Divalproex | CNS | Slightly |
| Dobutamine | Cardiovascular | Sparingly |
| Doxazosin | Cardiovascular | Slightly |
| Enalapril | Cardiovascular | Sparingly |
| Estradiol | Hormone | Insoluble |
| Etodolac | NSAID | Insoluble |
| Etoposide | Anticancer | Very Slightly |
| Famotidine | Gastrointestinal | Slightly |
| Felodipine | Cardiovascular | Insoluble |
| Fentanyl citrate | CNS | Sparingly |
| Fexofenadine | Respiratory | Slightly |
| Finasteride | Genito-urinary | Insoluble |
| Fluconazole | Antifungal | Slightly |
| Flunosolide | Respiratory | Insoluble |
| Flurbiprofen | NSAID | Slightly |
| Fluvoxamine | CNS | Sparingly |
| Furosemide | Cardiovascular | Insoluble |
| Glipizide | Metabolic | Insoluble |
| Glyburide | Metabolic | Sparingly |
| Ibuprofen | NSAID | Insoluble |

(continued)

| Poorly-Soluble Drug Candidates | | |
| --- | --- | --- |
| **Drug Name** | **Therapeutic Class** | **Solubility In Water** |
| Isosorbide dinitrate | Cardiovascular | Sparingly |
| Isotretinoin | Dermatological | Insoluble |
| Isradipine | Cardiovascular | Insoluble |
| Itraconzole | Antifungal | Insoluble |
| Ketoconazole | Antifungal | Insoluble |
| Ketoprofen | NSAID | Slightly |
| Lamotrigine | Class CNS | Slightly |
| Lansoprazole | Gastrointestinal | Insoluble |
| Loperamide | Gastrointestinal | Slightly |
| Loratadine | Respiratory | Insoluble |
| Lorazepam | CNS | Insoluble |
| Lovastatin | Cardiovascular | Insoluble |
| Medroxyprogesterone | Hormone | Insoluble |
| Mefenamic acid | Analgesic | Slightly |
| Methylprednisolone | Steroid | Insoluble |
| Midazolam | Anesthesia | Insoluble |
| Mometasone | Steroid | Insoluble |
| Nabumetone | NSAID | Insoluble |
| Naproxen | NSAID | Insoluble |
| Nicergoline | CNS | Insoluble |
| Nifedipine | Cardiovascular | Practically Insoluble |
| Norfloxacin | Anti-infective | Slightly |
| Omeprazole | Gastrointestinal | Slightly |
| Paclitaxel | Anticancer | Insoluble |
| Phenytoin | CNS | Insoluble |
| Piroxicam | NSAID | Sparingly |
| Quinapril | Cardiovascular | Insoluble |
| Ramipril | Cardiovascular | Insoluble |
| Risperidone | CNS | Insoluble |
| Saquinavir | Protease inhibitor | Practically insoluble |
| Sertraline | CNS | Slightly |
| Simvastatin | Cardiovascular | Insoluble |
| Terbinafine | Antifungal | Slightly |
| Terfenadine | Respiratory | Slightly |
| Triamcinolone | Steroid | Insoluble |
| Valproic acid | CNS | Slightly |
| Zolpidem | CNS | Sparingly |

Table 2

| Poorly-Soluble Drugs with Low Bioavailability | | | |
| --- | --- | --- | --- |
| **Drug Name** | **Indication** | **Solubility In Water** | **Bioavailability** |
| Astemizole | Allergic Rhinitis | Insoluble | Low - moderate |
| Cyclandelate | Peripheral vascular disease | Insoluble | Low |
| Perphenazine | Psychotic disorder | Insoluble | Low |

(continued)

| Poorly-Soluble Drugs with Low Bioavailability | | | |
|---|---|---|---|
| Drug Name | Indication | Solubility In Water | Bioavailability |
| Testosterone | Androgen Replacement Therapy | Insoluble | Low |
| Famotidine | GERD | Slightly soluble | Low (39-50%) |
| Budesonide | Allergic Rhinitis | Sparingly soluble | Low (~15%) |
| Mesalamine | Irritable Bowel Syndrome | Slightly soluble | Low (~20%) |
| Clemastine fumarate | Allergic Rhinitis | Slightly soluble | Low (~39%) |
| Buprenorphine | Pain | Slightly soluble | Low (<30%) |
| Sertraline | Anxiety | Slightly soluble | Low (<44%) |
| Auranofin | Arthritis | Slightly soluble | Low (15-25%) |
| Felodipine | Hypertension | Insoluble | Low (15%) |
| Isradipine | Hypertension | Insoluble | Low (15-24%) |
| Danazol | Endometriosis | Insoluble | Low |
| Loratadine | Allergic Rhinitis | Insoluble | Low |
| Isosorbide dinitrate | Angina | Sparingly soluble | Low (20-35%) |
| Fluphenazine | Psychotic disorder | Insoluble | Low (2-3%) |
| Spironolactone | Hypertension, Edema | Insoluble | Low (25%) |
| Biperiden | Parkinson's disease | Sparingly soluble | Low (29-33%) |
| Cyclosporin | Transplantation | Slightly soluble | Low (30%) |
| Norfloxacin | Bacterial Infection | Slightly soluble | Low (30-40%) |
| Cisapride | GERD | Insoluble | Low (35-40%) |
| Nabumetone | Arthritis | Insoluble | Low (35%) |
| Dronabinol | Antiemetic | Insoluble | Low 10-20%) |
| Lovastatin | Hyperlipidemia | Insoluble | Low (~5%) |
| Simvastatin | Hyperlipidemia | Insoluble | Low (<5%) |

An example of a low soluble drug to be loaded in a carrier according to the invention is a 15% (w/w) solution of Ziprasidone free base dissolved at 100°C in a liquefied mixture comprising roughly equal parts of a solvent, being either 2-pyrrolidone or 2-methyl pyrrolidone, and a high temperature melting solid, being either a lecithin, a polyvinylpyrrolidone polymer or a derivative hereof.

The amount of active substance incorporated in a tablet may be selected according to known principles of pharmaceutical formulation. In general, the dosage of the active substance present in a tablet according to the invention depends *inter alia* on the specific drug substance, the age and condition of the patient and of the disease to be treated.

In a specific embodiment of the invention the therapeutically, prophylactically and/or diagnostically active substance is solid at ambient temperature. However, this is not an absolute requirement, it may also be liquid at room temperature. The active substance may also be present in the form of a dispersion of the active substance in the pharmaceutically acceptable liquid formulation, or the active substance may be present in the form of an emulsion including a SMEDDs (self microemulsifying drug delivery system).

As mentioned above, the active substance may be dispersed in the pharmaceutically acceptable liquid formulation. In a specific embodiment, the active substance is at least partly dissolved in the pharmaceutically acceptable liquid formulation and/or it is at least partly present in an amorphous form. In one aspect of the invention the active substance may be present in amorphous form in the carrier at ambient temperature as measured by DSC.

**Other aspects of the invention**

The invention also relates to a method for the preparation of a tablet comprising the steps of:

i) preparation of a disintegrating loadable tablet as defined herein, optionally comprising one or more therapeutically, prophylactically and/or diagnostically active substances,

ii) loading the loadable tablet obtained from step i) with a pharmaceutically acceptable liquid formulation as defined in any of claims 33-59 optionally comprising one or more therapeutically, prophylactically and/or diagnostically active substances for at time period that is sufficient to saturate the loadable tablet with the pharmaceutically acceptable liquid formulation.

The loading of the loadable tablet with the pharmaceutically acceptable liquid formulation optionally comprising one or more therapeutically, prophylactically and/or diagnostically active substances is typically performed by spraying or it is performed by placing the loadable tablet in an excess of the pharmaceutically acceptable liquid formulation optionally comprising one or more therapeutically, prophylactically and/or diagnostically active substances.

In the method mentioned above, the time period in step ii) is normally at the most about 60 min such as, e.g., at the most 45 min or at the most 30 min for an amount of loadable tablets corresponding to 1 kg (and corresponding time periods for batches having another weight than 1 kg).

**List of Figures**

Figure 1A shows dissolution of a tablet according to the invention with and without gellan gum as described in Example 7

Figure 1B shows the dissolution of matrix before tabletting as described in Example 7

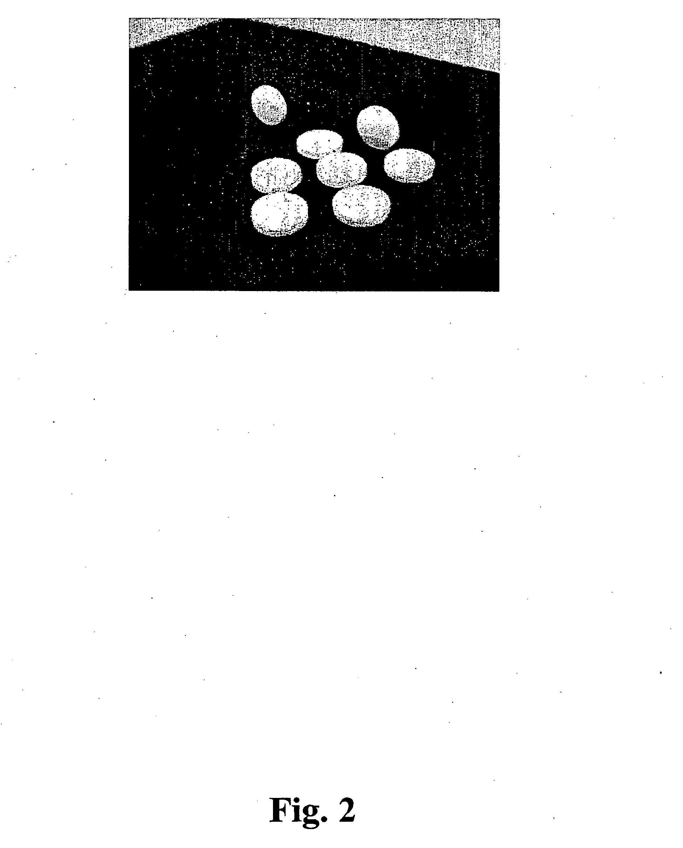

Figure 2 shows compressed loadable tablets comprising 99% Neusilin US2 and 1% magnesium stearate. The tablets has been produced on a Korch PH100 rotary tableting machine equipped with 12mm punches; rotations per minutes 39; tablets produced per minute 234; mean compression pressure 2.4 kN; mean hardness of tablets 91 N; mean weight of tablets 172.4 mg; mean porosity of tablets 78.5%. The produced tablets are uniform and suitable for further handling such as loading with a liquid.

The invention is further illustrated in the following non-limiting examples.

**Methods**

**Friability testing**

The friability of the loadable compressed tablets may be tested in accordance with USP, 1216 Tablet friability test or Ph. Eur. 2.9.7 Friability of uncoated tablets.

The apparatus meeting these specifications is available from laboratory supply houses such as VanKel Technology Group, 13000 Weston Parkway, Cary, NC 27513, or from Erweka Instruments, Inc., 56 Quirk Road, Milford, CT 06460.

Test procedure

For tablets with a unit mass equal to or less than 650 mg, take a sample of whole tablets corresponding to 6.5 g. For tablets with a unit mass of more than 650 mg, take a sample of 10 whole tablets. The tablets should be carefully dedusted prior to testing. Accurately weigh the tablet sample, and place the tablets in the drum. Rotate the drum 100 times with approx. 25 rpm, and remove the tablets. Remove any loose dust from the tablets as before, and accurately weight.

Generally, the test is run once. If obviously cracked, cleaved, or broken tablets are present in the tablet sample after tumbling, the sample fails the test. If the results are doubtful or if the weight loss is greater than the targeted value, the test should be repeated twice and the mean of the three tests determined.

A maximum mean weight loss from the three samples of not more than 1.0% is considered fully acceptable for the final dosage products.

However, according to the present invention, a higher weight loss may be acceptable for the loadable compressed tablet such as 1.5%, such as 2%, such as 2.5% may be acceptable as long as the loading capacity of the tablet allows for reproducible loading of the liquid.

**Examples**

**Example 1**

**Preparation of loadable tablets and properties thereof**

Six tablet compositions were manufactured based on the oil absorption materials Aeroperl 300 (Silicon dioxide, Degussa), Neusilin US2 (magnesium aluminium metasilicate, Fuji Chemical Industry) Avicel (microcrystalline cellulose, FMC) and Fujicalin SG, (dibasic calcium phosphate anhydrous, Fuji Chemical Industry).

| Composition 1 | |
|---|---|
| Neusilin US2 | 99% |
| Magnesium stearate | 1 % |

| Composition 2 | |
|---|---|
| Avicel PH102 | 99% |
| Magnesium stearate | 1% |

| Composition 3 | |
|---|---|
| Aeroperl 300 | 80% |
| PEG 6000 | 19% |
| Magnesium stearate | 1% |

| Composition 4 | |
|---|---|
| Aeroperl 300 | 55% |
| Avicel PH 101 | 44% |
| Magnesium stearate | 1 % |

| Composition 5 | |
|---|---|
| Avicel PH 102 | 99% |
| Magnesium stearate | 1 % |

| Composition 6 | |
|---|---|
| Fujicalin | 99% |
| Magnesium stearate | 1 % |

Magnesium stearate was blended with the remaining constituents in a Turbula blender for 3 minutes. The tablets were compressed on a single punch tabletting machine Diaf TM20. Tablet size: 9 mm round compound cup.

The tablets were placed in corn oil for 24 hours. The absorption of oil was completed within the first hour.

Tablets of composition 5 were loaded with with Imwitor 308, Sasol (glyceryl monocaprylate) with 10% dissolved Simvastatin. The loading with oil was performed at a temperature over melting point of Imwitor 308 (m.p. 35°C) correspondig to 40 °C.

Composition 1.

*Table 1. Oil absorption capacity of tablets containing Neusilin US2. (composition 1)*

| Tablet no. | Tablet core weight, mg | Tablet core incl. oil mg | Oil absorbed, mg | Oil absorbed % |
|---|---|---|---|---|
| 1 | 142 | 367 | 225 | 61.3 |
| 2 | 139 | 364 | 225 | 61.8 |
| 3 | 143 | 369 | 226 | 61.2 |
| 4 | 144 | 367 | 223 | 60.8 |
| 5 | 142 | 370 | 228 | 61.6 |
| 6 | 150 | 370 | 220 | 59.5 |
| Mean | 143 | 368 | 224.5 | 61.0 |

The tablet hardness was determined by Schleuninger 8M tablet hardness tester.

*Table 2. Tablet hardness before and after loading with oil (composition 1)*

| Mean tablet hardness before oil loading, N | Mean tablet hardness after oil loading, N |
|---|---|
| 38 | 34 |

The disintegration time was exceeding 24 hours before and after loading with oil. The disintegration time was decreased to less than 15 min. by addition of Ac-di-sol in a concentration of 1% (before loading) and reduced to 5 hours after oil loading. Ac-di-sol (croscarmellose sodium, FMC) is a superdisintegrant, which does not affect the oil absorption capacity of Neusilin.

The porosity of the tablets before loading is calculated on basis of the density of the tablet $\rho_t$ and the "true density" $\rho_s$ of the ingredients. The porosity $\varepsilon$ of the tablet is calculated according to the Equation 1.

$$\varepsilon = 1 - \frac{\rho_t}{\rho_s} \qquad \qquad \text{Equation 1.}$$

The density of the tablet is based on the ratio between weight and volume of the tablet. The "true density" of the ingredients is based on the gas pycnometrical density determined in helium using Micromeritics Accupyc 1330.

The maximum loading capacity of corn oil on weight basis is calculated according to Equation 2.

$$loading\ capcity\ w/w\% = \frac{\varepsilon}{\varepsilon + (1-\varepsilon)\frac{\rho_s}{\rho_l}} 100 \quad \text{Equation 2.}$$

The density of corn oil, $\rho_1 = 0.92$ g/ cm$^3$

*Table 3. Utilization of oil loading capacity (composition 1).*

| Porosity of the tablet % | Max. oil loading Capacity % | Measured oil loading % |
|---|---|---|
| 80 | 63 | 61 |

Composition 2

*Table 4. Oil absorption capacity of tablets with Avicel (composition 2)*

| Tablet no. | Tablet core weight, mg | Tablet core incl. oil mg | Oil absorbed mg | Oil absorbed % |
|---|---|---|---|---|
| 1 | 232 | 349 | 117 | 33.52 |
| 2 | 229 | 351 | 122 | 34.76 |
| 3 | 230 | 351 | 121 | 34.47 |
| 4 | 229 | 349 | 120 | 34.38 |
| 5 | 229 | 353 | 124 | 35.13 |
| 6 | 230 | 349 | 119 | 34.10 |
| Mean | 230 | 350 | 121 | 34.39 |

The tablet hardness is determined by Schleuninger 8M tablet hardness tester.

*Table 5. Tablet hardness before and after loading with oil (composition 2)*

| Mean tablet hardness before oil loading, N | Mean tablet hardness after oil loading, N |
|---|---|
| 33 | 32 |

*Table 6. Utilization of oil loading capacity (composition 2)*

| Porosity of the tablet % | Max. oil loading Capacity % | Measured oil loading % |
|---|---|---|
| 48 | 35 | 34 |

Composition 3

*Table 7. Oil absorption capacity of tablets with Aeroperl/PEG 6000 (composition 3)*

| Tablet no. | Tablet core weight, mg | Tablet core incl. oil mg | Oil absorbed, mg | Oil absorbed % |
|---|---|---|---|---|
| 1 | 105 | 222 | 117 | 52.7 |
| 2 | 108 | 226 | 118 | 52.2 |
| 3 | 113 | 230 | 117 | 50.9 |
| 4 | 106 | 228 | 122 | 53.5 |
| 5 | 126 | 232 | 106 | 45.7 |
| 6 | 110 | 227 | 117 | 51.5 |
| Mean | 111.3 | 227.5 | 116.2 | 51.1 |

The tablet hardness is determined by Schleuninger 8M tablet hardness tester.

*Table 8. Tablet hardness before and after loading with oil (composition 3)*

| Mean tablet hardness before oil loading, N | Mean tablet hardness after oil loading, N |
|---|---|
| 15 | 10 |

*Table 9. Utilization of oil loading capacity (composition 3)*

| Porosity of the tablet % | Max. oil loading Capacity % | Measured oil loading % |
|---|---|---|
| 70 | 54 | 51 |

Composition 4

*Table 10. Oil absorption capacity of tablets with Aeroperl/avicel (composition 4)*

| Tablet no. | Tablet core weight, mg | Tablet core incl. oil mg | Oil absorbed, mg | Oil absorbed % |
|---|---|---|---|---|
| 1 | 192 | 324 | 132 | 40.7 |
| 2 | 198 | 329 | 131 | 39.8 |
| 3 | 204 | 329 | 125 | 38.0 |
| 4 | 193 | 325 | 132 | 40.6 |
| 5 | 193 | 325 | 132 | 40.6 |
| Mean | 196 | 326 | 130 | 39.9 |

*Table 11. Tablet hardness before and after loading with oil (composition 4)*

| Mean tablet hardness before oil loading, N | Mean tablet hardness after oil loading, N |
|---|---|
| 30 | 27 |

*Table 12. Tablet disintegration time before and after loading with oil (composition 4)*

| Mean disintegration time before oil loading, min | Mean disintegration time after oil loading, min |
|---|---|
| 2 | 1 |

Compared to composition 3 the tabletting properties and tablet hardness were improved by addition of Avicel PH101 instead of PEG 6000.

Composition 5

*Table 13. Oil absorption capacity of tablets with A vicel. loaded with a 10% solution of Simvastatin in Imwitor 308. (composition 5)*

| Tablet no. | Tablet core weight, mg | Tablet core incl. oil mg | Oil absorbed, mg | Oil absorbed % |
|---|---|---|---|---|
| 1 | 229 | 338 | 109 | 32.2 |
| 2 | 229 | 337 | 108 | 32.0 |
| 3 | 229 | 337 | 108 | 32.0 |
| 4 | 229 | 339 | 110 | 32.4 |
| 5 | 230 | 338 | 108 | 31.9 |
| 6 | 229 | 337 | 108 | 32.0 |
| 7 | 229 | 338 | 109 | 32.2 |
| 8 | 229 | 338 | 109 | 32.2 |
| 9 | 229 | 339 | 110 | 32.4 |
| 10 | 228 | 339 | 111 | 32.7 |
| 11 | 230 | 340 | 110 | 32.4 |

(continued)

| Tablet no. | Tablet core weight, mg | Tablet core incl. oil mg | Oil absorbed, mg | Oil absorbed % |
|---|---|---|---|---|
| 12 | 230 | 338 | 108 | 31.9 |
| Mean | 229 | 338 | 109 | 32.2 |

*Table 14. Tablet hardness before and after loading with a 10% solution of Simvastatin in Imwitor 308 (composition 5)*

| Mean tablet hardness before oil loading, N | Mean tablet hardness after oil loading, N |
|---|---|
| 35 | 32 |

*Table 15. Tablet disintegration time before and after loading with oil (composition 5)*

| Mean disintegration time before oil loading, min | Mean disintegration time after oil loading, min |
|---|---|
| 1 | 2 |

Composition 6

*Table 17. Oil absorption capacity of tablets with Fujicalin loaded with corn oil (composition 6).*

| Tablet no. | Tablet core weight, mg | Tablet core incl. oil mg | Oil absorbed, mg | Oil absorbed % |
|---|---|---|---|---|
| 1 | 258 | 383 | 125 | 48.4 |
| 2 | 259 | 384 | 125 | 48.3 |
| 3 | 259 | 383 | 124 | 47.9 |
| 4 | 260 | 383 | 123 | 47.3 |
| 5 | 257 | 382 | 125 | 48.6 |
| 6 | 261 | 384 | 123 | 47.1 |
| Mean | 259 | 383.2 | 124.2 | 47.9 |

*Table 18. Tablet hardness before and after loading with corn oil (composition 6)*

| Mean tablet hardness before oil loading, N | Mean tablet hardness after oil loading, N |
|---|---|
| 42 | 20 |

*Table 19. Tablet disintegration time before and after loading with corn oil (composition 6)*

| Mean disintegration time before oil loading, min | Mean disintegration time after oil loading, min |
|---|---|
| 2 | 6.1 |

In order to investigate the influence of a disintegrant and optionally a hydrophilic substance on the absorption of oil, compositions were also prepared with a content of a superdisintegrant and, in some cases with a content of a sugar alcohol.

Thus, the following compositions have been prepared (% w/w)

| | | | | | | | | | | | | | | | | | | | | |
|---|---|---|---|---|---|---|---|---|---|---|---|---|---|---|---|---|---|---|---|---|
| **Neusilin US 2** | 94 | 94 | 94 | 94 | 89 | | | | | | | | | | | | | | | |
| **Avicel PH 102** | | | | | | 94 | 94 | 94 | 94 | 89 | | | | | | | | | | |
| **Aeroperl 300** | | | | | | | | | | | 94 | 94 | 94 | 94 | 89 | | | | | |
| **Fujicalin** | | | | | | | | | | | | | | | | 94 | 94 | 94 | 94 | 89 |
| **Ac-Di-Sol** | 2.5 | | | | | 2.5 | | | | | 2.5 | | | | | 2.5 | | | | |
| **Gellan gum LT 100** | | 2.5 | | 2.5 | 5 | | 2.5 | | 2.5 | 5 | | 2.5 | | 2.5 | 5 | | 2.5 | | 2.5 | 5 |
| **Kollidon CL** | | | 2.5 | | | | | 2.5 | | | | | 2.5 | | | | | 2.5 | | |
| **Xylitol** | 2.5 | 2.5 | 2.5 | | | | 2.5 | 2.5 | | | | 2.5 | 2.5 | | | | 2.5 | 2.5 | | |
| **Mannitol** | | | | 2.5 | 5 | 2.5 | | | 2.5 | 5 | 2.5 | | | 2.5 | 5 | 2.5 | | | 2.5 | 5 |
| **Magnesium Stearate** | 1 | 1 | 1 | 1 | 1 | 1 | 1 | 1 | 1 | 1 | 1 | 1 | 1 | 1 | 1 | 1 | 1 | 1 | 1 | 1 |

**Conclusion**

Porous tablets can be used as carriers for oily formulations such as oils, emulsions, microemulsions and semisolids liquefied at elevated temperature including drug substances as in liquid form or dissolved or dispersed in a liquid carrier. The oils can be applied to the tablets by conventional coating techniques (drums, perforated vessels or fluid bed). The feed rate of the oil should be adjusted to balance the rate of absorption of oil into the tablet cores.

The oil absorption capacity is determined by the porosity of the tablet core. The oil is filling the tablets voids close to saturation.

Any material, which provides tablets with porosities in the range of 30-90%, is applicable. Other materials than mentioned above may be applied as tablet core material, such as calcium carbonate, magnesium oxide preferable spray dried materials with satisfactory flowability and high specific surface area. The disintegration time of the tablets might be adjusted by addition of conventional tablet disintegrants and used in formulation of immediate release tablets as well as controlled release matrix tablets.

**Examples of porous tablets loaded with active substances (APIs)**

**Example 2**

Specification of core tablets

| | |
|---|---|
| Neusilin US2 | 93 mg |
| Magnesium stearate | 1 mg |
| Average tablet hardness: | 52 N |
| Tablet diameter: | 8 mm (compound cup) |

The tablets were compressed on a single punch tabletting machine Diaf TM20.

Specification of the loaded tablet (1 mg tacrolimus)

Tacrolimus in a concentration of 0.95% is dissolved in polyethylene glycol 400 and sprayed on Neusilin US2 core tablet at ambient temperature in a coating vessel. The composition of the loaded 1 mg tablet is shown in Table 1 corresponding to a loaded tablet weight of 200 mg corresponding to a load of vehicle of 53% w/w. Average tablet hardness: 52 N

Table 20. *Composition of a 1 mg tablet loaded with a solution of tacrolimus in PEG 400*

| Substance | mg |
|---|---:|
| Tacrolimus | 1.00 |
| PEG 400 | 105.0 |
| Neusilin US 2 | 93 |
| Magnesium stearate | 1 |
| Total | 200 |

A similar composition was prepared further containing 5 mg gellan gum and 2.5 mg xylitol.

**Example 3**

Specification of core tablets

| | |
|---|---|
| Neusilin US2 | 198 mg |

(continued)

| Magnesium stearate | 2 mg |
| Average tablet hardness: | 42 N |
| Tablet diameter: | 10 mm (compound cup) |

The tablets were compressed on a single punch tabletting machine Diaf TM20.

Specification of the loaded tablet (20 mg atorvastatin)

Atorvastatin in a concentration of 10 % is dissolved in melted Imwitor 308 (glyceryl monocaprylate) at 40°C and sprayed on Neusilin US2 core tablet heated to 35°C in a coating vessel. The loaded tablets are cooled in a refrigerator after loading in order to solidify the vehicle.

The composition of the loaded 20 mg tablet is shown in Table 2 corresponding to a loaded tablet weight of 400 mg corresponding to a load of vehicle of 50% w/w. Average tablet hardness: 48 N

Table 21. Composition of a 20 mg tablet loaded with a solution of atorvastatin in glyceryl monocaprylate.

| Substance | mg |
| --- | --- |
| Atorvastatin | 20.0 |
| Imwitor 308 | 180.0 |
| Neusilin US 2 | 198.0 |
| Magnesium stearate | 2.0 |
| Total | 400.0 |

A similar tablet was prepared further containing 5 mg Ac-Di-Sol.

**Example 4**

Specification of core tablets

| Neusilin US2 | 351 mg |
| Magnesium stearate | 2 mg |
| Average tablet hardness: | 60 N |
| Tablet shape: | Oblong tablet 9 x 19 mm |

The tablets were compressed on a single punch tableting machine Diaf TM20.

Specification of the loaded tablet (145 mg fenofibrate)

Fenofibrate in a concentration of 35 % is dissolved in a melted mixture of Polyethyleneglycol 6000 and Poloxamer 188 (70:30) at a temperature of 80°C and and sprayed on Neusilin US2 core tablet heated in a coating vessel to a temperature of 70°C. The tablets are cooled in the coating vessel after loading to a temperature below the melting point (60°C) of PEG and Poloxamer

The composition of the loaded 145 mg tablet is shown in Table 3 corresponding to a loaded tablet weight of 767 mg corresponding to a load of vehicle of 54% w/w. Average tablet hardness: 57 N

Table 22. Composition of a 145 mg tablet loaded with a solution of fenofibrate in a melted mixture of PEG 6000 and Poloxamer 188 (70:30).

| Substance | mg |
| --- | --- |
| Fenofibrate | 145.0 |

(continued)

| Substance | mg |
|---|---|
| PEG 6000 | 188.4 |
| Poloxamer 188 | 80.8 |
| Neusilin US 2 | 350.8 |
| Magnesium stearate | 2.0 |
| Total | 767.0 |

A similar tablet was prepared further containing 10 mg Kollidon CL and 10 mg xylitol.

**Example 5**

Specification of core tablets

| | |
|---|---|
| Neusilin US2 | 84 mg |
| Magnesium stearate | 1 mg |
| Average tablet hardness: | 42 N |
| Tablet diameter: | 7 mm (compound cup) |

The tablets were compressed on a single punch tableting machine Diaf TM20.

Specification of the loaded tablet (10 mq simvastatin)

Simvastatin in a concentration of 10 % is dissolved in (MCT) Viscoleo on Neusilin US2 core in a coating vessel. The composition of the loaded 10 mg tablet is shown in Table 4 corresponding to a loaded tablet weight of 185 mg corresponding to a load of vehicle of 54% w/w.

Table 23. Composition of a 10 mg tablet loaded with a solution of simvastatin in Viscoleo.

| Substance | mg |
|---|---|
| Simvastatin | 10.0 |
| Glyceryl monolaurate | 89.9 |
| Neusilin US 2 | 84.1 |
| Magnesium stearate | 1.0 |
| Total | 185.0 |

A similar tablet was prepared further containing 10 mg gellan gum and 10 mg xylitol.

**Example 6**

**Loading of neusilin tablets with Viscoleo (Medium chain glyceride)**

Tableffing process

Neusilin tablets were compressed on a single punch tabletting machine Diaf TM20:

Tablet properties before loading

Tablet diameter: 9 mm
Tablet shape: Compound cup
Tablet weight: 134 mg
Tablet weight variation, $S_{rel}$ : 1.6%
Tablet hardness: 51 N (determined on hardness tester Schleuniger M8)

Loading process (loading process)

50 g tablets were loaded with viscoleo in a lab-scale fluid bed Phast FB 100 using a coating module with top-spray
Atomization air flow: 1 $m^3$ per hour
Fluidization air flow: 40 $m^3$ per hour
Liquid feed rate: 2.5 g min
Coating time until saturation of the tablets: 30 min.
Weight increase: 67.5 g viscoleo.

Tablet properties after loading

Tablet weight: 305 mg (loading 56 w/w%)
Tablet hardness: 51 N
Tablet weight variation, $S_{rel}$: 5.1 %

Conclusion

Conventional coating equipment as a fluid bed is feasible for loading a liquid formulation on the porous tablets within a short processing time. The tablets quickly absorb the liquid applied by spraying on the tablet surface. The tablet hardness is not affected by the loading with the liquid. The weight variation is increased from 1.6% to 5.2% still being within acceptable limits related to dose variation when an active substance is incorporated.

**Example 7**

**Release of Ibuprofen from a loaded tablet**

Ibuprofen in rape seed oil carrier loaded into a neusilin tablet is compared with a similar tablet also comprising gellan gum and xylitol.
The release is tested by dissolution according to the USP 2 method in buffer at pH 7.2 and ibuprofen is measured by UV spectrophotometer (wavelength absorption at abs 222nm)

Material and methods

*Table 24 material and methods*

| Substance | Batch no. | Function | Supplier/manufacturer |
|---|---|---|---|
| Neusilin US2 | 0504004 | Matrix | |
| Xylitol | 0403038 | Absorption of water into tablet | UNIKEM |
| Gellan gum LT 100 | 0509034 | Disintegrating | Cpkelco |
| Mg-stearate | 0403110 | Tabletting | |
| EtOH | - | Granulation wetting | |
| Ibuprofen | 373191 | API | UNIKEM |
| Rape seed oil | - | Carrier for API | |

*Table 24 dissolution parameters*

| Substance | Function | Supplier/manufacturer |
|---|---|---|
| Buffer pH 7.2 | Dissolution medie | |
| ibuprofen | Standard | UNIKEM |

Equipment

Tabletting DIAF             No. U-TABD-02
Mixer turbola
Heating plate
Punch 9mm round         No. Life 0004
VanKel apparatus 2       No.. a-DISA-003
Spectrofotometric Shimadzu   No. A-spek-01

Procedure:

Composition of tablets without gellan gum

*Table 25 tablet composition*

| Substance | Batch no. | % | Weighted |
|---|---|---|---|
| Neusilin US2 | 0504004 | 99 | 49,5 |
| Mg-stearate | 0403110 | 1 | 0,5 |

Composition of tablets with gellan gum

*Table 26 tablet composition*

| Substance | Batch no. | % | Weighted g |
|---|---|---|---|
| Neusilin US2 | 0504004 | 89 | 44,5 |
| Xylitol | 0403038 | 5 | 2,5 |
| Gellan gum LT 100 | 0509034 | 5 | 2,5 |
| Mg-stearate | 0403110 | 1 | 0,5 |
| EtOH | | | |

Granulate comprising disintegrant

Xylitol and Gellan gum are mixed with addition of a small amount of EtOH, Neusilin US2 are added and the blend is mixed in Turbola mixer for 3 min. Mg-stearate is added to the blend and mixed in additional 1/2 min in the Turbola mixer.

Tabletting:

Both tablet types (with and without disintegrant) are prepared on the DIAF with a weight of approx. 145 mg and with a hardness of approx 50N

Loading of the ibuprofen composition:

*Table 27 carrier/API composition*

| Substance | % | Weighted g |
|---|---|---|
| Rape seed oil | 80 | 4.0 |
| Ibuprofen | 20 | 1.0 |

Ibuprofen is mixed with the rape seed oil during heating to approx. 40°C until a homogeneous mass/solution. The neusilin tablets are added to the liquid and is left for approximately 2h until fully loaded (saturated) with the API/ carrier solution. The final weight is then about 420 mg per tablet.

Dissolution:

Temp. 37°C
Rpm. 75/min
Medium: Buffer pH 7.2, 900 ml.
Sample times: every ½h for 5h,

Calculation of release

The ibuprofen standard is prepared with ibuprofen in buffer at pH 7.2 and measured spectometric at a wavelength of 222 nm. For the calculation of the released amount it is contemplated that the tablets are loaded with 54 mg ibuprofen for the tablets comprising gellan gum and 64 mg when the tablets are without gellan gum. The ibuprofen is calculated as a 100% pure API. The dissolution curve demonstrates that ibuprofen, a low solubility drug has a very slow release from the loaded tablet.

Results:

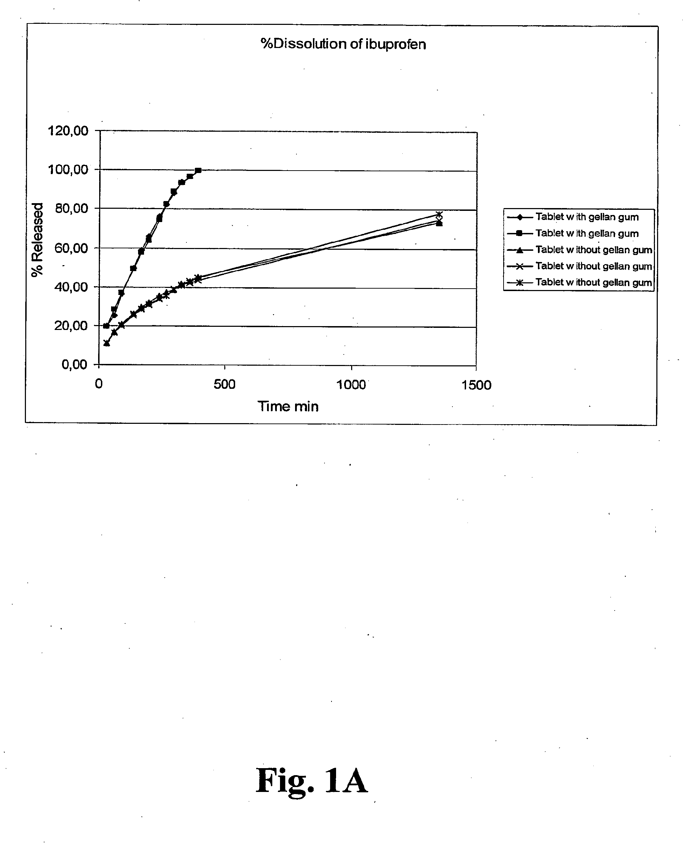

It is clear from the release profiles disclosed in Figure 1A that the release of the low soluble drug ibuprofen is distinctly increased when gellan gum and xylitol is included into the composition. The dissolution time for approximately 80% release is decreased from about 1400 minutes to about 270 minutes corresponding to approximately 80% decrease in dissolution time.

*Table 28 release measured by dissolution*

| % released | Tablet without Gellan gum | Tablet with gellan gum | % of time for approximate similar release by use of gellan gum |
|---|---|---|---|
| 25 (26) | 140 min | 60 min | 43% |
| 50 (48) | 390 min | 140 min | 35% |
| 75 | 1350 | 200 (from curve) | 15% |
| 80 (82) | 1400 (from curve) | 270 min | 20% |
| 100 | Not measurable | 360 | |

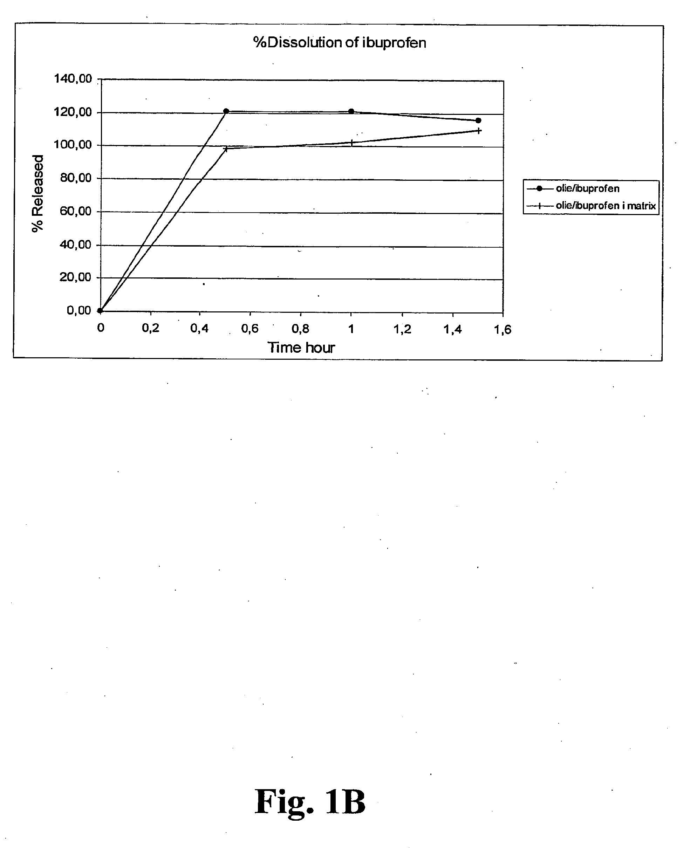

Figure 1B demonstrates the different in release from the matrix (i.e. a mixture of all ingredients contained in the loaded tablet) before tabletting in order to demonstrate that the difference in release with and without gellan gum is not related to any influence from the tabletting process.

**Example 8**

**Disintegration time for different superdisintegrants**

| | |
|---|---|
| Batch size: | 100 g |
| Punch diameter: | 9 mm |
| Mixing equipment: | Turbula 3 min og ½ min. |
| Hardness: | Approx. 100 N |

To 100 g of the mixture of Emcompress (dicalcium phosphate) with 1 % magnesium stearate, the disintegrant is added in the amounts disclosed below.

*Table 29 disintegration time for different binder/disintegrant tablet compositions*

| Binder, g | Disintegrant, g | Hardness, N average | Mg-stearate G | Disintegration time Ph. Eur |
|---|---|---|---|---|
| Emcompress, 99 | None | 108 | 1 | Above 60 min. |
| Emcompress 99 | Kollidon CL, 1 | 97 | 1 | 28 sec |
| Emcompress 99,5 | Kollidon CL 0,5 | 104 | 1 | 34 sec |
| Emcompress 99,5 | Ac-Di-Sol ,0,5 | 115 | 1 | 31 sec |

**Example 9**

**Test of gellan gum as superdisintegrant**

| | |
|---|---|
| Batch size: | 100 g |
| Punch diameter: | 9 mm |
| Mizing equipment: | Turbula 3 min og ½ min. |
| Hardness: | Approx. 100 N |

To 100 g of the mixture of Emcompress (dicalcium phosfate) with 1% magnesium stearate, Gellan gum is added in the amounts disclosed below.

*Table 30 disintegration of different binder/Gellan gum tablet compositions*

| Binder, g | Disintegrant, g | Hardness, N average | Mg-stearate G | Disintegration time Ph. Eur |
|---|---|---|---|---|
| Emcompress, 99 | None | 108 | 1 | Above |
| Emcompress 99,5 | Gellan gum, 0,5 | 107 | 1 | 34 sec |
| Emcompress 99 | Gellan gum, 1 | 99 | 1 | 24 sec |

This Example demonstrates that Gellan gum has surprisingly high disintegrating, properties similar to or even higher than disintegrants known as superdisintegrants which compared to gellan gum have demonstrated insufficient integrating properties in the loadable tablets according to the invention.

### Example 10

**Estimated disintegrating times for different carriers used for loading the tablet**

Tablets were prepared with the following ingredients:

| | |
|---|---|
| Neusilin | 44.5 mg |
| Gellan gum | 2.5 mg |
| Xylitol | 2.5 mg |
| Mg stearate | 0.5 mg |

The tablets were loaded with one of the vehicles stated in the table below by placing the tablets in each vehicle until saturation.

The estimated disintegrating time is based on visual inspection and in tap water at ambient temperature, i.e. the tablets are dropped into a glass of tap water and the time it takes to disintegrate the tablets into primary particles is noted as the estimated disintegration time. As appears from the Table, carriers having high water solubility (Gelucire/PEG 400) demonstrate a longer disintegration time than the oily substances. Accordingly, the skilled person can select a suitable carrier in accordance with a specific desired disintegration/release. The disintegration can then be further improved with disintegrants or mixtures according to the present invention.

*Table 31 disintegration of tablets loaded with different carriers*

| Vehicle | Estimated disintegrating time |
|---|---|
| PEG 400 | Approx. 4 h |
| Gelucire 44/14 | Aprox 4 |
| Gelucie 50/13 | 4 h |
| Glycerol (85%) | 45 min |
| Propylenglycol | 45 min |
| Rape seed oil | 10 min |
| Myglyol 812N | 5 min |

### Example 11

**Test of effect on different amount disintegrant on disintegrating time (tested according to Ph.Eur.)**

*Table 32 disintegration time for different amounts of* same *disintegrant*

| Disintegrant Ac-di-sol Content in unloaded tablets (%) | Hardness | Disintegration time buffer pH6.8 | Disintegration time HCL (0.1 N) | |
|---|---|---|---|---|
| 1 | 38 | 3.5h | 2h | |
| 1.5 | 39 | 10min | 28 min | |
| 2 | 46 | 38 sec | 34sec | |

As appears from the table the amount of disintegrant can be used for controlling the disintegrating rates.

### Example 12

**Test of loaded tablets from Example 11**

*Table 33 disintegration if different carriers with different amount* of *same disintegrant*

| Disintegrant (%) | Loaded with 100% PEG 400 | Loaded with 100% propylenglycol | Disintegration in HCL (0.1N), Ph. Eur. |
|---|---|---|---|
| 1 | | + | No disintegration for 3.5h |
| 1.5 | + | | 1 h 20 min |
| 1.5 | | + | 1 h 22 min |
| 2 | + | | 1 h 53 min |
| 2 | | + | 1h 22 min |

**Claims**

1. A disintegrating loadable tablet product in compressed form solely containing inert pharmaceutically acceptable excipients, the tablet comprising

    i) at least 60% w/w of a sorbent material selected from metal oxides and metal silicates having a specific surface area (BET surface area) of at least 50 m2/g as measured by gas adsorption or mixtures of such sorbent materials, and which - when manufactured into tablets together with at the most 50% w/w of lactose - provide a tablet that has a porosity of 45 vol % or more,
    ii) a disintegrant or a mixture of disintegrants

    wherein the tablet in compressed form has

    a) a porosity of 45% v/v or more,
    b) a hardness of at least 20 Newton, and
    c) a loading capacity of at least 30% of a liquid.

2. A disintegrating loadable tablet according to claim 1, wherein the metal of said metal oxide or metal silicate is selected from the group consisting of sodium, potassium, magnesium, calcium, zink, aluminium, titanium and silicium.

3. A disintegrating loadable tablet according to claim 1 or 2, wherein the sorbent material is a metal oxide selected from the group consisting of magnesium oxide, calcium oxide, zink oxide, aluminium oxide, titanium dioxide including Tronox A-HP-328 and Tronox A-HP-100, silicium dioxides including Aerosil, Cab-O-Sil, Syloid, Aeroperl, Aeroperl 300, Sunsil (silicon beads), Zeofree, Sipernat, Zeopharm S170, Zeopharm 6000, and mixtures thereof.

4. A disintegrating loadable tablet according to claim 3, wherein the metal oxide is a titanium dioxide or a silicium dioxide or mixtures thereof.

5. A disintegrating loadable tablet according to claim 3 or 4, wherein the metal oxide is , a non-porous silicate including fumed silicas of the Aerosil type.

6. A disintegrating loadable tablet according to claim 3 or 4, wherein the metal oxide is a porous silicate including e.g. Syloid, Porasil and Lichrosorp.

7. A disintegrating loadable tablet according to any of the preceding claims, wherein the sorbent material is a metal silicate selected from the group consisting of sodium silicate, potassium silicate, magnesium silicate, calcium silicate including synthetic calcium silicate such as, e.g., Hubersorp, zink silicate, aluminum silicate, sodium aluminosilicate such as, e.g., Zeolex, magnesium aluminum silicate, magnesium aluminum metasilicate, aluminium metasilicate, such as Neusilin S1, Neusilin SN2N, Neusilin SG2, Neusilin US2, and mixtures thereof.

8. A disintegrating loadable tablet according to any of the preceding claims, wherein the metal silicate is selected from alkaline earth metal silicates and aluminum silicates included magnesium aluminum metasilicate.

9. A disintegrating loadable tablet according to claim 7 or 8, wherein the metal silicate is Neusilin US2.

10. A disintegrating loadable tablet according to any of the preceding claims, wherein the disintegrant is a superdisintegrant.

11. A disintegrating loadable tablet according to claim 10, wherein the superdisintegrant is selected from the group consisting of sodium carboxymethyl cellulose (Ac-Di-Sol®, Clod-2®), Crosslinked polyvinylpyrrolidone (Polyplasdone-X1 R, Polyplasdone-XL 10R, Kollidon-CLR) and gellan gum.

12. A disintegrating loadable tablet according to any of claims 1-9, wherein the disintegrant is selected from the group consisting of formalin-casein, L-HPC, chitin, chitosan, polymerized agar acrylamide, xylan, smecta, key-jo-clay, crosslinked carboxymethylguar and modified tapioca starch, alginic acid or alginates, microcrystalline cellulose, hydroxypropyl cellulose and other cellulose derivatives, croscarmellose sodium, crospovidone, polacrillin potassium, sodium starch glycolate, starch, pregelatinized starch, carboxymethyl starch (e.g. Primogel® and Explotab®) etc.

13. A disintegrating loadable tablet according to any of the preceding claims, wherein the concentration of the disintegrant in the tablet is from about 0.5% to about 15% w/w such as, e.g., from about 0.1% w/w to 15 % w/w, such as from about 0.2% to 10% w/w, such as from 0.3 to 8% w/w, such as from 0.4 to 8% w/w such as 0.5 to 5% w/w, or from about 1% to about 10% w/w, from about 1% to about 8% w/w, from about 1% to about 5% w/w, from about 1% to about 3% w/w or from about 0.5 to about 5% w/w.

14. A method for the preparation of a disintegrating loadable tablet according to any of claims 1-13 comprising the steps of:

i) mixing the at least 60% w/w of said sorbent material with said disintegrant or mixture of disintegrants and optionally further pharmaceutically acceptable excipients,
ii) compressing the solid mixture to form tablets with a hardness of in a range of from 20N to 150N.

15. A method according to claim 14 further comprising a step of loading the disintegrating loadable tablet obtained according to claim 14 with a pharmaceutically acceptable liquid formulation to a concentration of about 35% w/w or more (based on the total weight of the loaded tablet) optionally comprising one or more therapeutically, prophylactically and/or diagnostically active substances for at time period that is sufficient to saturate the disintegrating loadable tablet with the pharmaceutically acceptable liquid formulation.

**Patentansprüche**

1. Zerfallendes befüllbares Tablettenprodukt in komprimierter Form, weiches ausschließlich inerte pharmazeutisch verträgliche Exzipienten enthält, wobei die Tablette umfasst:

i) wenigstens 60 % Gew./Gew. von einem Sorbensmaterial, ausgewählt aus Metalloxiden und Metallsilicaten mit einer spezifischen Oberfläche (BET-Oberfläche) von wenigstens 50 m$^2$/g, gemessen durch Gasadsorption, oder Mischungen von solchen Sorbensmaterialien, und welche - wenn sie zusammen mit höchstens 50 % Gew./Gew. Lactose zu Tabletten verarbeitet werden - eine Tablette ergeben, welche eine Porosität von 45 Vol,-% oder mehr aufweist,
ii) ein Sprengmittel bzw. Zerfallhilfsmittel oder eine Mischung von Sprengmitteln,

wobei die Tablette in komprimierter Form

a) eine Porosität von 45 % Vol/Vol. oder mehr,
b) eine Härte von wenigstens 20 Newton, und
c) eine Befüllungskapazität bzw. ein Aufnahmevermögen von wenigstens 30 % einer Flüssigkeit aufweist.

2. Zerfallende befüllbare Tablette nach Anspruch 1, wobei das Metall des Metalloxids oder Metallsilicats ausgewählt ist aus der Gruppe bestehend aus Natrium, Kalium, Magnesium, Calcium, Zink, Aluminium, Titan und Silicium.

3. Zerfallende befüllbare Tablette nach Anspruch 1 oder 2, wobei das Sorbensmaterial ein Metalloxid ist, das ausgewählt ist aus der Gruppe bestehend aus Magnesiumoxid, Calciumoxid, Zinkoxid, Aluminiumoxid, Titandioxid, einschließlich Tronox A-HP-328 und Tronox A-HP-100, Siliciumdioxiden, einschließlich Aerosil, Cab-O-Sil, Syloid, Aeroperl, Aeroperl 300, Sunsil (Siliciumperlen), Zeofree, Sipernat, Zeopharm S170, Zeopharm 6000 und Mischungen davon.

4. Zerfallende befüllbare Tablette nach Anspruch 3, wobei das Metalloxid ein Titandioxid oder ein Siliciumdioxid oder Mischungen davon ist.

5. Zerfallende befüllbare Tablette nach Anspruch 3 oder 4, wobei das Metalloxid ein nichtporöses Silicat, einschließlich pyrogener Kieselsäuren vom Aerosil-Typ, ist.

6. Zerfallende befüllbare Tablette nach Anspruch 3 oder 4, wobei das Metalloxid ein poröses Silicat, einschließlich z. B. Syloid, Porasil und Lichrosorp, ist.

7. Zerfallende befüllbare Tablette nach einem der vorangehenden Ansprüche, wobei das Sorbensmaterial ein Metallsilicat ist, das ausgewählt ist aus der Gruppe bestehend aus Natriumsilicat, Kaliumsilicat, Magnesiumsilicat, Calciumsilicat, einschließlich synthetischen Calciumsilicats wie z. B. Hubersorp, Zinksilicat, Aluminiumsilicat, Natriumaluminosilicat wie z. B. Zeolex, Magnesiumaluminiumsilicat, Magnesiumaluminiummetasilicat, Aluminiummetasilicat wie Neusilin S1, Neusilin SN2N, Neusilin SG2, Neusilin US2 und Mischungen davon.

8. Zerfallende befüllbare Tablette nach einem der vorangehenden Ansprüche, wobei das Metallsilicat ausgewählt ist aus Erdalkalimetallsilicaten und Aluminiumsilicaten, einschließlich Magnesiumaluminiummetasilicat.

9. Zerfallende befüllbare Tablette nach Anspruch 7 oder 8, wobei das Metallsilicat Neusilin US2 ist.

10. Zerfallende befüllbare Tablette nach einem der vorangehenden Ansprüche, wobei das Sprengmittel ein Supersprengmittel ist.

11. Zerfallende befüllbare Tablette nach Anspruch 10, wobei das Supersprengmittel ausgewählt ist aus der Gruppe bestehend aus Natriumcarboxymethylcellulose (Ac-Di-Sol®, CLD-2®), vernetztem Polyvinylpyrrolidon (Polyp)asdone-X1R, Polypiasdone-XL 10R, Kollidon-CLR) und Gellangummi.

12. Zerfallende befüllbare Tablette nach einem der Ansprüche 1-9, wobei das Sprengmittel ausgewählt ist aus der Gruppe bestehend aus Formalin-Casein, L-HPC, Chitin, Chitosan, polymerisiertem Agaracrylamid, Xylan, Smecta, Key-jo-Clay, vernetztem Carboxymethylguar und modifizierter Tapiokastärke, Alginsäure oder Alginaten, mikrokristalliner Cellulose, Hydroxypropylcellulose und anderen Cellulosederivaten, Croscarmellose-Natrium, Crospovidon, Polacrillin-Kalium, Natriumstärkeglycolat, Stärke, vorgelatinisierter Stärke, Carboxymethylstärke (z. B. Primogel® und Explotab®) usw.

13. Zerfallende befüllbare Tablette nach einem der vorangehenden Ansprüche, wobei die Konzentration des Sprengmittels in der Tablette ungefähr 0,5 % bis ungefähr 15 % Gew./Gew., wie etwa z. B. ungefähr 0,1 % Gew./Gew. bis 15 % Gew./Gew., wie etwa ungefähr 0,2 % bis 10 % Gew./Gew., wie etwa 0,3 bis 8 % Gew./Gew., wie etwa 0,4 bis 8 % Gew./Gew., wie etwa 0,5 bis 5 % Gew./Gew. oder ungefähr 1 % bis ungefähr 10 % Gew./Gew., ungefähr 1 % bis ungefähr 8 % Gew./Gew., ungefähr 1 % bis ungefähr 5 % Gew./Gew., ungefähr 1 % bis ungefähr 3 % Gew./Gew. oder ungefähr 0,5 bis ungefähr 5 % Gew./Gew. beträgt.

14. Verfahren zur Herstellung einer zerfallenden befüllbaren Tablette nach einem der Ansprüche 1-13, umfassend die Schritte:

    i) Mischen der wenigstens 60 % Gew./Gew. des Sorbensmaterials mit dem Sprengmittel oder der Mischung von Sprengmitteln und gegebenenfalls weiteren pharmazeutisch verträglichen Exzipienten,
    ii) Komprimieren der festen Mischung, um Tabletten mit einer Härte in einem Bereich von 20 N bis 150 N zu bilden.

15. Verfahren nach Anspruch 14, außerdem umfassend einen Schrift des Befüllens- der zerfallenden befüllbaren Tablette, die gemäß Anspruch 14 erhalten wurde, mit einer pharmazeutisch verträglichen flüssigen Formulierung bis zu einer Konzentration von ungefähr 35 % Gew./Gew. oder mehr (bezogen auf das Gesamtgewicht der befüllten Tablette), gegebenenfalls umfassend eine oder mehrere therapeutisch, prophylaktisch und/oder diagnostisch wirksame Substanzen, über einen Zeitraum, weicher ausreicht, um die zerfallende befüllbare Tablette mit der pharmazeutisch verträglichen flüssigen Formulierung zu sättigen.

**Revendications**

1. Produit en comprimé chargeable désintégrable sous forme compressée contenant uniquement des excipients inertes pharmaceutiquement acceptables, le comprimé comprenant

   i) au moins 60 % p/p d'un matériau sorbant choisi parmi les oxydes métalliques et les silicates métalliques ayant une surface spécifique (surface spécifique BET) d'au moins 50 m$^2$/g telle que mesurée par adsorption de gaz ou de mélanges de tels matériaux sorbants et qui, lorsqu'ils sont fabriqués en comprimés conjointement avec au plus 50 % p/p de lactose, donnent un comprimé qui a une porosité de 45 % en volume ou plus,
   ii) un délitant ou un mélange de délitants,

   lequel comprimé, sous forme compressée, a

   a) une porosité de 45 % v/v ou plus,
   b) une dureté d'au moins 20 Newtons, et
   c) une capacité de chargement d'au moins 30 % d'un liquide.

2. Comprimé chargeable désintégrable selon la revendication 1, dans lequel le métal dudit oxyde métallique ou silicate métallique est choisi dans le groupe constitué par le sodium, le potassium, le magnésium, le calcium, le zinc, l'aluminium, le titane et le silicium.

3. Comprimé chargeable désintégrable selon la revendication 1 ou 2, dans lequel le matériau sorbant est un oxyde métallique choisi dans l'ensemble constitué par l'oxyde de magnésium, l'oxyde de calcium, l'oxyde de zinc, l'oxyde d'aluminium, le dioxyde de titane, y compris Tronox A-HP-328 et Tronox A-HP-100, les dioxydes de silicium, y compris Aerosil, Cab-O-Sil, Syloid, Aeroperl, Aeroperl 300, Sunsil (perles de silicium), Zeofree, Sipernat, Zeopharm S170, Zeopharm 6000, et leurs mélanges.

4. Comprimé chargeable désintégrable selon la revendication 3, dans lequel l'oxyde métallique est un dioxyde de titane ou un dioxyde de silicium ou un de leurs mélanges.

5. Comprimé chargeable désintégrable selon la revendication 3 ou 4, dans lequel l'oxyde métallique est un silicate non poreux, y compris les silices fumées du type Aerosil.

6. Comprimé chargeable désintégrable selon la revendication 3 ou 4, dans lequel l'oxyde métallique est un silicate poreux, y compris, par exemple, Syloid, Porasil et Lichrosorp.

7. Comprimé chargeable désintégrable selon l'une quelconque des revendications précédentes, dans lequel le matériau sorbant est un silicate métallique choisi dans l'ensemble constitué par un silicate de sodium, un silicate de potassium, un silicate de magnésium, un silicate de calcium, y compris un silicate de calcium synthétique comme, par exemple Hubersorp, un silicate de zinc, un silicate d'aluminium, un silicate d'aluminium et de sodium comme par exemple, Zeolex, un silicate d'aluminium et de magnésium, un métasilicate d'aluminium et de magnésium, un métasilicate d'aluminium, tel que Neusilin S1, Neusilin SN2N, Neusilin SG2, Neusilin US2, et leurs mélanges.

8. Comprimé chargeable désintégrable selon l'une quelconque des revendications précédentes, dans lequel le silicate métallique est choisi parmi les silicates de métal alcalino-terreux et les silicates d'aluminium, y compris un métasilicate d'aluminium et de magnésium.

9. Comprimé chargeable désintégrable selon la revendication 7 ou 8, dans lequel le silicate métallique est le Neusilin US2.

10. Comprimé chargeable désintégrable selon l'une quelconque des revendications précédentes, dans lequel le délitant est un super-délitant.

11. Comprimé chargeable désintégrable selon la revendication 10, dans lequel le super-délitant est choisi dans l'ensemble constitué par la carboxyméthylcellulose sodique (Ac-Di-Sol®, CLD-2®), la polyvinylpyrrolidone réticulée (Polyplasdone-X1R, Polyplasdone-XL 10R, Kollidon-CLR) et la gomme gellane.

12. Comprimé chargeable désintégrable selon l'une quelconque des revendications 1 à 9, dans lequel le délitant est choisi dans l'ensemble constitué par la formaline-caséine, L-HPC, la chitine, le chitosane, un acrylamide de gélose polymérisé, le xylane, l'argile smecta, l'argile kay-jo, le carboxyméthyl-guar réticulé et l'amidon de tapioca modifié, l'acide alginique et les alginates, la cellulose microcristalline, l'hydroxypropylcellulose et d'autres dérivés de cellulose, la croscarmellose sodique, la crospovidone, la polacrilline potassique, le glycolate d'amidon sodique, l'amidon, l'amidon pré-gélatinisé, le carboxyméthyl-amidon (par exemple Primogel® et Explotab®), etc.

13. Comprimé chargeable désintégrable selon l'une quelconque des revendications précédentes, dans lequel la concentration du délitant dans le comprimé est d'environ 0,5 % à environ 15 % p/p, comme par exemple d'environ 0,1 % p/p à 15 % p/p, comme d'environ 0,2 % à 10 % p/p, comme de 0,3 à 8 % p/p, comme de 0, 4 à 8 % p/p, comme de 0, 5 à 5 % p/p, ou d'environ 1 % % à environ 10 % p/p, d'environ 1 % à environ 8 % p/p, d'environ 1 % à environ 5 % p/p, d'environ 1 % à environ 3 % p/p ou d'environ 0,5 à environ 5 % p/p.

14. Procédé pour la préparation d'un comprimé chargeable désintégrable de l'une quelconque des revendications 1 à 13, comprenant les étapes consistant à :

   i) mélanger les au moins 60 % p/p dudit matériau sorbant avec ledit délitant ou mélange de délitants et éventuellement d'autres excipients pharmaceutiquement acceptables,
   ii) compresser le mélange solide pour former des comprimés ayant une dureté située dans la plage allant de 20 N à 150 N.

15. Procédé selon la revendication 14, comprenant en outre une étape consistant à charger le comprimé chargeable désintégrable obtenu conformément à la revendication 14 avec une formulation liquide pharmaceutiquement acceptable jusqu'à une concentration d'environ 35 % p/p ou plus (sur la base du poids total du comprimé chargé), comprenant éventuellement une ou plusieurs substances ayant une activité thérapeutique, prophylactique et/ou diagnostique pendant une période de temps suffisante pour saturer le comprimé chargeable désintégrable avec la formulation liquide pharmaceutiquement acceptable.

**Fig. 1A**

Fig. 1B

**Fig. 2**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 03004001 A
- US 20020086055 A
- WO 0038655 A
- WO 002005051358 A
- US 6399591 B
- US 20050019398 A
- US 20040253312 A
- WO 03063831 A
- WO 0050007 A